# EUROPEAN PATENT APPLICATION

(11) **EP 2 591 796 A1**
(43) Date of publication of application: **15.05.2013**
(21) Application number: 11188625.5
(22) Date of filing: 10.11.2011
(51) Int. Cl.: A61K 38/20, A61K 39/395, A61K 45/06, A61P 35/00

(54) **Combination medicament comprising IL-12 and an anti-CTLA-4 ligand for tumor therapy**

(71) Applicant: Universität Zürich, 8006 Zürich (CH)
(72) Inventor: Becher, Burkhard, Dr., Prof., CH-8124 Maur (CH)
(74) Representative: Schulz Junghans

(57) **Abstract**

The invention relates to a combination medicament for treatment of malignant neoplastic disease. The combination medicament comprises an IL-12 polypeptide having a biological activity of IL-12 or a nucleic acid expression vector comprising a sequence encoding such IL-12 polypeptide, and a non-agonist CTLA-4 ligand, particularly an anti-CTLA-4 immunoglobulin G.

SEQUENCE LISTING

<110>  Universitaet Zuerich

<120>  Combination medicament comprising IL-12 and an anti-CTLA-4 ligand
        for tumor therapy

<130>  uz117ep-1

<160>  9

<170>  PatentIn version 3.5

<210>  1
<211>  775
<212>  PRT
<213>  artificial

<220>
<223>  fusion between human IL-12 and IgG4 Fc

<400>  1

Met Cys His Gln Gln Leu Val Ile Ser Trp Phe Ser Leu Val Phe Leu
1               5                   10                  15

Ala Ser Pro Leu Val Ala Ile Trp Glu Leu Lys Lys Asp Val Tyr Val
            20                  25                  30

Val Glu Leu Asp Trp Tyr Pro Asp Ala Pro Gly Glu Met Val Val Leu
        35                  40                  45

Thr Cys Asp Thr Pro Glu Glu Asp Gly Ile Thr Trp Thr Leu Asp Gln
    50                  55                  60

Ser Ser Glu Val Leu Gly Ser Gly Lys Thr Leu Thr Ile Gln Val Lys
65                  70                  75                  80

Glu Phe Gly Asp Ala Gly Gln Tyr Thr Cys His Lys Gly Gly Glu Val
                85                  90                  95

Leu Ser His Ser Leu Leu Leu Leu His Lys Lys Glu Asp Gly Ile Trp
            100                 105                 110

Ser Thr Asp Ile Leu Lys Asp Gln Lys Glu Pro Lys Asn Lys Thr Phe
        115                 120                 125

Leu Arg Cys Glu Ala Lys Asn Tyr Ser Gly Arg Phe Thr Cys Trp Trp
    130                 135                 140

Leu Thr Thr Ile Ser Thr Asp Leu Thr Phe Ser Val Lys Ser Ser Arg
145                 150                 155                 160

Gly Ser Ser Asp Pro Gln Gly Val Thr Cys Gly Ala Ala Thr Leu Ser
                165                 170                 175

Ala Glu Arg Val Arg Gly Asp Asn Lys Glu Tyr Glu Tyr Ser Val Glu
            180                 185                 190

Cys Gln Glu Asp Ser Ala Cys Pro Ala Ala Glu Glu Ser Leu Pro Ile
        195                 200                 205

Glu Val Met Val Asp Ala Val His Lys Leu Lys Tyr Glu Asn Tyr Thr
    210                 215                 220

Ser Ser Phe Phe Ile Arg Asp Ile Ile Lys Pro Asp Pro Pro Lys Asn
225                 230                 235                 240

Leu Gln Leu Lys Pro Leu Lys Asn Ser Arg Gln Val Glu Val Ser Trp
                245                 250                 255

Glu Tyr Pro Asp Thr Trp Ser Thr Pro His Ser Tyr Phe Ser Leu Thr
            260                 265                 270

Phe Cys Val Gln Val Gln Gly Lys Ser Lys Arg Glu Lys Lys Asp Arg
        275                 280                 285

Val Phe Thr Asp Lys Thr Ser Ala Thr Val Ile Cys Arg Lys Asn Ala
    290                 295                 300

Ser Ile Ser Val Arg Ala Gln Asp Arg Tyr Tyr Ser Ser Ser Trp Ser
305                 310                 315                 320

Glu Trp Ala Ser Val Pro Cys Ser Gly Gly Gly Gly Ser Gly Gly Gly
                325                 330                 335

Gly Ser Gly Gly Gly Gly Ser Arg Asn Leu Pro Val Ala Thr Pro Asp
            340                 345                 350

Pro Gly Met Phe Pro Cys Leu His His Ser Gln Asn Leu Leu Arg Ala
        355                 360                 365

Val Ser Asn Met Leu Gln Lys Ala Arg Gln Thr Leu Glu Phe Tyr Pro
    370                 375                 380

Cys Thr Ser Glu Glu Ile Asp His Glu Asp Ile Thr Lys Asp Lys Thr
385                 390                 395                 400

Ser Thr Val Glu Ala Cys Leu Pro Leu Glu Leu Thr Lys Asn Glu Ser
                405                 410                 415

Cys Leu Asn Ser Arg Glu Thr Ser Phe Ile Thr Asn Gly Ser Cys Leu
            420                 425                 430

Ala Ser Arg Lys Thr Ser Phe Met Met Ala Leu Cys Leu Ser Ser Ile
        435                 440                 445

Tyr Glu Asp Leu Lys Met Tyr Gln Val Glu Phe Lys Thr Met Asn Ala
    450                 455                 460

Lys Leu Leu Met Asp Pro Lys Arg Gln Ile Phe Leu Asp Gln Asn Met
465                 470                 475                 480

Leu Ala Val Ile Asp Glu Leu Met Gln Ala Leu Asn Phe Asn Ser Glu
                485                 490                 495

Thr Val Pro Gln Lys Ser Ser Leu Glu Glu Pro Asp Phe Tyr Lys Thr
            500                 505                 510

Lys Ile Lys Leu Cys Ile Leu Leu His Ala Phe Arg Ile Arg Ala Val
        515                 520                 525

Thr Ile Asp Arg Val Met Ser Tyr Leu Asn Ala Ser Lys Val Asp Lys
    530                 535                 540

Arg Val Glu Ser Lys Tyr Gly Pro Pro Cys Pro Ser Cys Pro Ala Pro
545                 550                 555                 560

Glu Phe Leu Gly Gly Pro Ser Val Phe Leu Phe Pro Pro Lys Pro Lys
                565                 570                 575

Asp Thr Leu Met Ile Ser Arg Thr Pro Glu Val Thr Cys Val Val Val
            580                 585                 590

Asp Val Ser Gln Glu Asp Pro Glu Val Gln Phe Asn Trp Tyr Val Asp
        595                 600                 605

Gly Val Glu Val His Asn Ala Lys Thr Lys Pro Arg Glu Glu Gln Phe
    610                 615                 620

Asn Ser Thr Tyr Arg Val Val Ser Val Leu Thr Val Leu His Gln Asp
625                 630                 635                 640

Trp Leu Asn Gly Lys Glu Tyr Lys Cys Lys Val Ser Asn Lys Gly Leu
                645                 650                 655

Pro Ser Ser Ile Glu Lys Thr Ile Ser Lys Ala Lys Gly Gln Pro Arg
            660                 665                 670

Glu Pro Gln Val Tyr Thr Leu Pro Pro Ser Pro Glu Glu Met Thr Lys
        675                 680                 685

Asn Gln Val Ser Leu Thr Cys Leu Val Lys Gly Phe Tyr Pro Ser Asp
    690                 695                 700

Ile Ala Val Glu Trp Glu Ser Asn Gly Gln Pro Glu Asn Asn Tyr Lys
705                 710                 715                 720

Thr Thr Pro Pro Val Leu Asp Ser Asp Gly Ser Phe Phe Leu Tyr Ser
                725                 730                 735

Arg Leu Thr Val Asp Lys Ser Arg Trp Gln Glu Gly Asn Val Phe Ser
            740                 745                 750

Cys Ser Val Met His Glu Ala Leu His Asn His Tyr Thr Gln Lys Ser
        755                 760                 765

Leu Ser Leu Ser Leu Gly Lys
    770                 775

<210>  2
<211>  781
<212>  PRT
<213>  Artificial Sequence

<220>
<223>  murine IL-12 IgG3 Fc fusion construct

<400>  2

Met Cys Pro Gln Lys Leu Thr Ile Ser Trp Phe Ala Ile Val Leu Leu
1               5                   10                  15

Val Ser Pro Leu Met Ala Met Trp Glu Leu Glu Lys Asp Val Tyr Val
            20                  25                  30

Val Glu Val Asp Trp Thr Pro Asp Ala Pro Gly Glu Thr Val Asn Leu
        35                  40                  45

Thr Cys Asp Thr Pro Glu Glu Asp Asp Ile Thr Trp Thr Ser Asp Gln
    50                  55                  60

Arg His Gly Val Ile Gly Ser Gly Lys Thr Leu Thr Ile Thr Val Lys
65                  70                  75                  80

Glu Phe Leu Asp Ala Gly Gln Tyr Thr Cys His Lys Gly Gly Glu Thr
                85                  90                  95

Leu Ser His Ser His Leu Leu Leu His Lys Lys Glu Asn Gly Ile Trp
            100                 105                 110

Ser Thr Glu Ile Leu Lys Asn Phe Lys Asn Lys Thr Phe Leu Lys Cys
        115                 120                 125

Glu Ala Pro Asn Tyr Ser Gly Arg Phe Thr Cys Ser Trp Leu Val Gln
    130                 135                 140

Arg Asn Met Asp Leu Lys Phe Asn Ile Lys Ser Ser Ser Ser Ser Pro
145                 150                 155                 160

Asp Ser Arg Ala Val Thr Cys Gly Met Ala Ser Leu Ser Ala Glu Lys
                165                 170                 175

Val Thr Leu Asp Gln Arg Asp Tyr Glu Lys Tyr Ser Val Ser Cys Gln
            180                 185                 190

Glu Asp Val Thr Cys Pro Thr Ala Glu Glu Thr Leu Pro Ile Glu Leu
        195                 200                 205

Ala Leu Glu Ala Arg Gln Gln Asn Lys Tyr Glu Asn Tyr Ser Thr Ser
    210                 215                 220

Phe Phe Ile Arg Asp Ile Ile Lys Pro Asp Pro Pro Lys Asn Leu Gln
225                 230                 235                 240

Met Lys Pro Leu Lys Asn Ser Gln Val Glu Val Ser Trp Glu Tyr Pro
                245                 250                 255

Asp Ser Trp Ser Thr Pro His Ser Tyr Phe Ser Leu Lys Phe Phe Val
            260                 265                 270

Arg Ile Gln Arg Lys Lys Glu Lys Met Lys Glu Thr Glu Glu Gly Cys
        275                 280                 285

Asn Gln Lys Gly Ala Phe Leu Val Glu Lys Thr Ser Thr Glu Val Gln
    290                 295                 300

Cys Lys Gly Gly Asn Val Cys Val Gln Ala Gln Asp Arg Tyr Tyr Asn
305                 310                 315                 320

Ser Ser Cys Ser Lys Trp Ala Cys Val Pro Cys Arg Val Arg Ser Gly
                325                 330                 335

Gly Gly Gly Ser Gly Gly Gly Gly Ser Gly Gly Gly Gly Ser Arg Val
            340                 345                 350

Ile Pro Val Ser Gly Pro Ala Arg Cys Leu Ser Gln Ser Arg Asn Leu
        355                 360                 365

Leu Lys Thr Thr Asp Asp Met Val Lys Thr Ala Arg Glu Lys Leu Lys
    370                 375                 380

His Tyr Ser Cys Thr Ala Glu Asp Ile Asp His Glu Asp Ile Thr Arg
385                 390                 395                 400

Asp Gln Thr Ser Thr Leu Lys Thr Cys Leu Pro Leu Glu Leu His Lys
                405                 410                 415

Asn Glu Ser Cys Leu Ala Thr Arg Glu Thr Ser Ser Thr Thr Arg Gly
            420                 425                 430

Ser Cys Leu Pro Pro Gln Lys Thr Ser Leu Met Met Thr Leu Cys Leu
        435                 440                 445

Gly Ser Ile Tyr Glu Asp Leu Lys Met Tyr Gln Thr Glu Phe Gln Ala
    450                 455                 460

Ile Asn Ala Ala Leu Gln Asn His Asn His Gln Gln Ile Ile Leu Asp
465                 470                 475                 480

Lys Gly Met Leu Val Ala Ile Asp Glu Leu Met Gln Ser Leu Asn His
                485                 490                 495

Asn Gly Glu Thr Leu Arg Gln Lys Pro Pro Val Gly Glu Ala Asp Pro
            500                 505                 510

Tyr Arg Val Lys Met Lys Leu Cys Ile Leu Leu His Ala Phe Ser Thr
        515                 520                 525

Arg Val Val Thr Ile Asn Arg Val Met Gly Tyr Leu Ser Ser Ala Leu
    530                 535                 540

Ile Lys Arg Ile Glu Pro Arg Ile Pro Lys Pro Ser Thr Pro Pro Gly
545                 550                 555                 560

Ser Ser Cys Pro Pro Gly Asn Ile Leu Gly Gly Pro Ser Val Phe Ile
                565                 570                 575

Phe Pro Pro Lys Pro Lys Asp Ala Leu Met Ile Ser Leu Thr Pro Lys
            580                 585                 590

Val Thr Cys Val Val Val Asp Val Ser Glu Asp Asp Pro Asp Val His
        595                 600                 605

Val Ser Trp Phe Val Asp Asn Lys Glu Val His Thr Ala Trp Thr Gln
    610                 615                 620

Pro Arg Glu Ala Gln Tyr Asn Ser Thr Phe Arg Val Val Ser Ala Leu
625                 630                 635                 640

Pro Ile Gln His Gln Asp Trp Met Arg Gly Lys Glu Phe Lys Cys Lys

                645                 650                 655

Val Asn Asn Lys Ala Leu Pro Ala Pro Ile Glu Arg Thr Ile Ser Lys
            660                 665                 670

Pro Lys Gly Arg Ala Gln Thr Pro Gln Val Tyr Thr Ile Pro Pro Pro
        675                 680                 685

Arg Glu Gln Met Ser Lys Lys Lys Val Ser Leu Thr Cys Leu Val Thr
    690                 695                 700

Asn Phe Phe Ser Glu Ala Ile Ser Val Glu Trp Glu Arg Asn Gly Glu
705                 710                 715                 720

Leu Glu Gln Asp Tyr Lys Asn Thr Pro Pro Ile Leu Asp Ser Asp Gly
                725                 730                 735

Thr Tyr Phe Leu Tyr Ser Lys Leu Thr Val Asp Thr Asp Ser Trp Leu
            740                 745                 750

Gln Gly Glu Ile Phe Thr Cys Ser Val Val His Glu Ala Leu His Asn
        755                 760                 765

His His Thr Gln Lys Asn Leu Ser Arg Ser Pro Gly Lys
    770                 775                 780

<210> 3
<211> 17
<212> DNA
<213> artificial

<220>
<223> PCR primer

<400> 3
acacacagcc tggacgc 17

<210> 4
<211> 20
<212> DNA
<213> artificial

<220>
<223> PCR primer

<400> 4
catttgaact ccttgcccct 20

<210> 5
<211> 219
<212> PRT
<213> homo sapiens

<400> 5

Met Cys Pro Ala Arg Ser Leu Leu Leu Val Ala Thr Leu Val Leu Leu
1               5                   10                  15

Asp His Leu Ser Leu Ala Arg Asn Leu Pro Val Ala Thr Pro Asp Pro
            20                  25                  30

Gly Met Phe Pro Cys Leu His His Ser Gln Asn Leu Leu Arg Ala Val
        35                  40                  45

Ser Asn Met Leu Gln Lys Ala Arg Gln Thr Leu Glu Phe Tyr Pro Cys
    50                  55                  60

Thr Ser Glu Glu Ile Asp His Glu Asp Ile Thr Lys Asp Lys Thr Ser
65                  70                  75                  80

Thr Val Glu Ala Cys Leu Pro Leu Glu Leu Thr Lys Asn Glu Ser Cys
                85                  90                  95

Leu Asn Ser Arg Glu Thr Ser Phe Ile Thr Asn Gly Ser Cys Leu Ala
            100                 105                 110

Ser Arg Lys Thr Ser Phe Met Met Ala Leu Cys Leu Ser Ser Ile Tyr
        115                 120                 125

Glu Asp Leu Lys Met Tyr Gln Val Glu Phe Lys Thr Met Asn Ala Lys
    130                 135                 140

Leu Leu Met Asp Pro Lys Arg Gln Ile Phe Leu Asp Gln Asn Met Leu
145                 150                 155                 160

Ala Val Ile Asp Glu Leu Met Gln Ala Leu Asn Phe Asn Ser Glu Thr
                165                 170                 175

Val Pro Gln Lys Ser Ser Leu Glu Glu Pro Asp Phe Tyr Lys Thr Lys
            180                 185                 190

Ile Lys Leu Cys Ile Leu Leu His Ala Phe Arg Ile Arg Ala Val Thr
        195                 200                 205

Ile Asp Arg Val Met Ser Tyr Leu Asn Ala Ser
    210                 215

<210>  6
<211>  328
<212>  PRT
<213>  homo sapiens

<400>  6

Met Cys His Gln Gln Leu Val Ile Ser Trp Phe Ser Leu Val Phe Leu

1               5                   10                  15

Ala Ser Pro Leu Val Ala Ile Trp Glu Leu Lys Lys Asp Val Tyr Val
            20                  25                  30

Val Glu Leu Asp Trp Tyr Pro Asp Ala Pro Gly Glu Met Val Val Leu
        35                  40                  45

Thr Cys Asp Thr Pro Glu Glu Asp Gly Ile Thr Trp Thr Leu Asp Gln
    50                  55                  60

Ser Ser Glu Val Leu Gly Ser Gly Lys Thr Leu Thr Ile Gln Val Lys
65                  70                  75                  80

Glu Phe Gly Asp Ala Gly Gln Tyr Thr Cys His Lys Gly Gly Glu Val
                85                  90                  95

Leu Ser His Ser Leu Leu Leu Leu His Lys Lys Glu Asp Gly Ile Trp
            100                 105                 110

Ser Thr Asp Ile Leu Lys Asp Gln Lys Glu Pro Lys Asn Lys Thr Phe
        115                 120                 125

Leu Arg Cys Glu Ala Lys Asn Tyr Ser Gly Arg Phe Thr Cys Trp Trp
    130                 135                 140

Leu Thr Thr Ile Ser Thr Asp Leu Thr Phe Ser Val Lys Ser Ser Arg
145                 150                 155                 160

Gly Ser Ser Asp Pro Gln Gly Val Thr Cys Gly Ala Ala Thr Leu Ser
                165                 170                 175

Ala Glu Arg Val Arg Gly Asp Asn Lys Glu Tyr Glu Tyr Ser Val Glu
            180                 185                 190

Cys Gln Glu Asp Ser Ala Cys Pro Ala Ala Glu Glu Ser Leu Pro Ile
        195                 200                 205

Glu Val Met Val Asp Ala Val His Lys Leu Lys Tyr Glu Asn Tyr Thr
    210                 215                 220

Ser Ser Phe Phe Ile Arg Asp Ile Ile Lys Pro Asp Pro Pro Lys Asn
225                 230                 235                 240

Leu Gln Leu Lys Pro Leu Lys Asn Ser Arg Gln Val Glu Val Ser Trp
                245                 250                 255

Glu Tyr Pro Asp Thr Trp Ser Thr Pro His Ser Tyr Phe Ser Leu Thr
            260                 265                 270

Phe Cys Val Gln Val Gln Gly Lys Ser Lys Arg Glu Lys Lys Asp Arg
        275                 280                 285

Val Phe Thr Asp Lys Thr Ser Ala Thr Val Ile Cys Arg Lys Asn Ala
    290                 295                 300

Ser Ile Ser Val Arg Ala Gln Asp Arg Tyr Tyr Ser Ser Ser Trp Ser
305                 310                 315                 320

Glu Trp Ala Ser Val Pro Cys Ser
                325

<210>  7
<211>  2328
<212>  DNA
<213>  artificial

<220>
<223>  expression construct, coding sequence for IL-12 IgG4 Fc fusion
       protein

<400>  7
atgtgtcacc agcagttggt catctcttgg ttttccctgg tttttctggc atctcccctc      60

gtggccatat gggaactgaa gaaagatgtt tatgtcgtag aattggattg gtatccggat     120

gcccctggag aaatggtggt cctcacctgt gacacccctg aagaagatgg tatcacctgg     180

accttggacc agagcagtga ggtcttaggc tctggcaaaa ccctgaccat ccaagtcaaa     240

gagtttggag atgctggcca gtacacctgt cacaaaggag gcgaggttct aagccattcg     300

ctcctgctgc ttcacaaaaa ggaagatgga atttggtcca ctgatatttt aaaggaccag     360

aaagaaccca aaaataagac ctttctaaga tgcgaggcca agaattattc tggacgtttc     420

acctgctggt ggctgacgac aatcagtact gatttgacat tcagtgtcaa aagcagcaga     480

ggctcttctg acccccaagg ggtgacgtgc ggagctgcta cactctctgc agagagagtc     540

agaggggaca acaaggagta tgagtactca gtggagtgcc aggaggacag tgcctgccca     600

gctgctgagg agagtctgcc cattgaggtc atggtggatg ccgttcacaa gctcaagtat     660

gaaaactaca ccagcagctt cttcatcagg gacatcatca aacctgaccc acccaagaac     720

ttgcagctga agccattaaa gaattctcgg caggtggagg tcagctggga gtaccctgac     780

acctggagta ctccacattc ctacttctcc ctgacattct gcgttcaggt ccagggcaag     840

agcaagagag aaaagaaaga tagagtcttc acggacaaga cctcagccac ggtcatctgc     900

cgcaaaaatg ccagcattag cgtgcgggcc caggaccgct actatagctc atcttggagc     960

gaatgggcat ctgtgccctg cagtggaggc ggtggctcgg gcggtggtgg gtcgggtggc    1020

ggcggatcca gaaacctccc cgtggccact ccagacccag gaatgttccc atgccttcac    1080

cactcccaaa acctgctgag ggccgtcagc aacatgctcc agaaggccag acaaactcta    1140

gaattttacc cttgcacttc tgaagagatt gatcatgaag atatcacaaa agataaaacc 1200

agcacagtgg aggcctgttt accattggaa ttaaccaaga atgagagttg cctaaattcc 1260

agagagacct ctttcataac taatgggagt tgcctggcct ccagaaagac ctcttttatg 1320

atggccctgt gccttagtag tatttatgaa gacttgaaga tgtaccaggt ggagttcaag 1380

accatgaatg caaagcttct gatggatcct aagaggcaga tctttctaga tcaaaacatg 1440

ctggcagtta ttgatgagct gatgcaggcc ctgaatttca acagtgagac tgtgccacaa 1500

aaatcctccc ttgaagaacc ggatttttat aaaactaaaa tcaagctctg catacttctt 1560

catgctttca gaattcgggc agtgactatt gatagagtga tgagctatct gaatgcttcc 1620

aaggtggaca agagagttga gtccaaatat ggtcccccat gcccatcatg cccagcacct 1680

gagttcctgg ggggaccatc agtcttcctg ttccccccaa aacccaagga cactctcatg 1740

atctcccgga cccctgaggt cacgtgcgtg gtggtggacg tgagccagga agaccccgag 1800

gtccagttca actggtacgt ggatggcgtg gaggtgcata atgccaagac aaagccgcgg 1860

gaggagcagt tcaacagcac gtaccgtgtg gtcagcgtcc tcaccgtcct gcaccaggac 1920

tggctgaacg gcaaggagta caagtgcaag gtctccaaca aaggcctccc gtcctccatc 1980

gagaaaacca tctccaaagc caaagggcag ccccgagagc cacaggtgta caccctgccc 2040

ccatccccgg aggagatgac caagaaccag gtcagcctga cctgcctggt caaaggcttc 2100

taccccagcg acatcgccgt ggagtgggag agcaatgggc agccggagaa caactacaag 2160

accacgcctc ccgtgctgga ctccgacggc tccttcttcc tctacagcag gctaaccgtg 2220

gacaagagca ggtggcagga ggggaatgtc ttctcatgct ccgtgatgca tgaggctctg 2280

cacaaccact acacacagaa gagcctctcc ctgtctctgg gtaaatga 2328

<210> 8
<211> 10994
<212> DNA
<213> artificial

<220>
<223> plasmid vector encoding Fc tag (murine)

<400> 8
   ctcgcagcaa agcaagatgt gtcctcagaa gctaaccatc tcctggtttg ccatcgtttt 60
   gctggtgtct ccactcatgg ccatgtggga gctggagaag cttatcaaga gaatcgagcc 120
   tagaataccc aagcccagta cccccccagg ttcttcatgc ccacctggta acatcttggg 180
   tggaccatcc gtcttcatct tccccccaaa gcccaaggat gcactcatga tctccctaac 240
   ccccaaggtt acgtgtgtgg tggtggatgt gagcgaggat gacccagatg tccatgtcag 300
   ctggtttgtg gacaacaaag aagtacacac agcctggacg cagccccgtg aagctcagta 360
   caacagtacc ttccgagtgg tcagtgccct ccccatccag caccaggact ggatgagggg 420
   caaggagttc aaatgcaagg tcaacaacaa agccctccca gcccccatcg agagaaccat 480
   ctcaaaaccc aaaggaagag cccagacacc tcaagtatac accatacccc cacctcgtga 540
   acaaatgtcc aagaagaagg ttagtctgac ctgcctggtc accaacttct tctctgaagc 600
   catcagtgtg gagtgggaaa ggaacggaga actggagcag gattacaaga acactccacc 660
   catcctggac tcggatggga cctacttcct ctacagcaag ctcactgtgg atacagacag 720
   ttggttgcaa ggagaaattt ttacctgctc cgtggtgcat gaggctctcc ataaccacca 780
   cacacagaag aacctgtctc gctcccctgg taaatgagaa cagcatctag cggccgctcg 840
   aggccggcaa ggccggatcc agacatgata agatacattg atgagtttgg acaaaccaca 900
   actagaatgc agtgaaaaaa atgctttatt tgtgaaattt gtgatgctat tgctttattt 960
   gtaaccatta taagctgcaa taaacaagtt aacaacaaca attgcattca ttttatgttt 1020
   caggttcagg gggaggtgtg ggaggttttt taaagcaagt aaaacctcta caaatgtggt 1080
   atggctgatt atgatccggc tgcctcgcgc gtttcggtga tgacggtgaa aacctctgac 1140
   acatgcagct cccggagacg gtcacagctt gtctgtaagc ggatgccggg agcagacaag 1200
   cccgtcaggc gtcagcgggt gttggcgggt gtcggggcgc agccatgagg tcgactctag 1260
   aggatcgatg ccccgccccg gacgaactaa acctgactac gacatctctg ccccttcttc 1320
   gcggggcagt gcatgtaatc ccttcagttg gttggtacaa cttgccaact gggccctgtt 1380
   ccacatgtga cacggggggg gaccaaacac aaaggggttc tctgactgta gttgacatcc 1440
   ttataaatgg atgtgcacat ttgccaacac tgagtggctt tcatcctgga gcagactttg 1500
   cagtctgtgg actgcaacac aacattgcct ttatgtgtaa ctcttggctg aagctcttac 1560
   accaatgctg ggggacatgt acctcccagg ggcccaggaa gactacggga ggctacacca 1620
   acgtcaatca gaggggcctg tgtagctacc gataagcgga ccctcaagag ggcattagca 1680
   atagtgttta taaggccccc ttgttaaccc taaacgggta gcatatgctt cccgggtagt 1740
   agtatatact atccagacta accctaattc aatagcatat gttacccaac gggaagcata 1800
   tgctatcgaa ttagggttag taaaagggtc ctaaggaaca gcgatatctc ccaccccatg 1860
   agctgtcacg gttttattta catggggtca ggattccacg agggtagtga accattttag 1920
   tcacaagggc agtggctgaa gatcaaggag cgggcagtga actctcctga atcttcgcct 1980
   gcttcttcat tctccttcgt ttagctaata gaataactgc tgagttgtga acagtaaggt 2040
   gtatgtgagg tgctcgaaaa caaggtttca ggtgacgccc ccagaataaa atttggacgg 2100
   ggggttcagt ggtggcattg tgctatgaca ccaatataac cctcacaaac cccttgggca 2160
   ataaatacta gtgtaggaat gaaacattct gaatatcttt aacaatagaa atccatgggg 2220
   tggggacaag ccgtaaagac tggatgtcca tctcacacga atttatggct atgggcaaca 2280
   cataatccta gtgcaatatg atactggggt tattaagatg tgtcccaggc agggaccaag 2340
   acaggtgaac catgttgtta cactctattt gtaacaaggg gaaagagagt ggacgccgac 2400
   agcagcggac tccactggtt gtctctaaca cccccgaaaa ttaaacgggg ctccacgcca 2460
   atggggccca taaacaaaga caagtggcca ctcttttttt tgaaattgtg gagtgggggc 2520
   acgcgtcagc ccccacacgc cgccctgcgg ttttggactg taaaataagg gtgtaataac 2580
   ttggctgatt gtaaccccgc taaccactgc ggtcaaacca cttgcccaca aaaccactaa 2640
   tggcaccccg gggaatacct gcataagtag gtgggcgggc caagataggg gcgcgattgc 2700
   tgcgatctgg aggacaaatt acacacactt gcgcctgagc gccaagcaca gggttgttgg 2760
   tcctcatatt cacgaggtcg ctgagagcac ggtgggctaa tgttgccatg ggtagcatat 2820
   actacccaaa tatctggata gcatatgcta tcctaatcta tatctgggta gcataggcta 2880
   tcctaatcta tatctgggta gcatatgcta tcctaatcta tatctgggta gtatatgcta 2940
   tcctaattta tatctgggta gcataggcta tcctaatcta tatctgggta gcatatgcta 3000
   tcctaatcta tatctgggta gtatatgcta tcctaatctg tatccgggta gcatatgcta 3060
   tcctaataga gattagggta gtatatgcta tcctaattta tatctgggta gcatatacta 3120
   cccaaatatc tggatagcat atgctatcct aatctatatc tgggtagcat atgctatcct 3180
   aatctatatc tgggtagcat aggctatcct aatctatatc tgggtagcat atgctatcct 3240
   aatctatatc tgggtagtat atgctatcct aatttatatc tgggtagcat aggctatcct 3300
   aatctatatc tgggtagcat atgctatcct aatctatatc tgggtagtat atgctatcct 3360
   aatctgtatc cgggtagcat atgctatcct catgcatata cagtcagcat atgataccca 3420
   gtagtagagt gggagtgcta tcctttgcat atgccgccac ctcccaaggg ggcgtgaatt 3480
   ttcgctgctt gtccttttcc tgctggttgc tcccattctt aggtgaattt aaggaggcca 3540
   ggctaaagcc gtcgcatgtc tgattgctca ccaggtaaat gtcgctaatg ttttccaacg 3600
   cgagaaggtg ttgagcgcgg agctgagtga cgtgacaaca tgggtatgcc caattgcccc 3660
   atgttgggag gacgaaaatg gtgacaagac agatggccag aaatacacca acagcacgca 3720
   tgatgtctac tggggattta ttctttagtg cgggggaata cacggctttt aatacgattg 3780
   agggcgtctc ctaacaagtt acatcactcc tgcccttcct caccctcatc tccatcacct 3840
   ccttcatctc cgtcatctcc gtcatcaccc tccgcggcag ccccttccac cataggtgga 3900
   aaccagggag gcaaatctac tccatcgtca aagctgcaca cagtcaccct gatattgcag 3960
   gtaggagcgg gctttgtcat aacaaggtcc ttaatcgcat ccttcaaaac ctcagcaaat 4020
   atatgagttt gtaaaaagac catgaaataa cagacaatgg actcccttag cgggccaggt 4080
   tgtgggccgg gtccaggggc cattccaaag gggagacgac tcaatggtgt aagacgacat 4140
   tgtggaatag caagggcagt tcctcgcctt aggttgtaaa gggaggtctt actacctcca 4200
   tatacgaaca caccggcgac ccaagttcct tcgtcggtag tcctttctac gtgactccta 4260
   gccaggagag ctcttaaacc ttctgcaatg ttctcaaatt tcgggttgga acctccttga 4320
   ccacgatgct ttccaaacca ccctcctttt ttgcgcctgc ctccatcacc ctgaccccgg 4380
   ggtccagtgc ttgggccttc tcctgggtca tctgcggggc cctgctctat cgctcccggg 4440
   ggcacgtcag gctcaccatc tgggccacct tcttggtggt attcaaaata atcggcttcc 4500
   cctacagggt ggaaaaatgg ccttctacct ggagggggcc tgcgcggtgg agacccggat 4560
   gatgatgact gactactggg actcctgggc ctcttttctc cacgtccacg acctctcccc 4620
   ctggctcttt cacgacttcc ccccctggct ctttcacgtc ctctaccccg gcggcctcca 4680
   ctacctcctc gaccccggcc tccactacct cctcgacccc ggcctccact gcctcctcga 4740
   ccccggcctc cacctcctgc tcctgcccct cctgctcctg cccctcctcc tgctcctgcc 4800
   cctcctgccc ctcctgctcc tgcccctcct gcccctcctg ctcctgcccc tcctgcccct 4860
   cctgctcctg cccctcctgc ccctcctcct gctcctgccc ctcctgcccc tcctcctgct 4920
   cctgcccctc ctgcccctcc tgctcctgcc cctcctgccc ctcctgctcc tgcccctcct 4980
   gcccctcctg ctcctgcccc tcctgctcct gcccctcctg ctcctgcccc tcctgctcct 5040
   gcccctcctg cccctcctgc ccctcctcct gctcctgccc ctcctgctcc tgcccctcct 5100
   gcccctcctg cccctcctgc tcctgcccct cctcctgctc ctgcccctcc tgcccctcct 5160
   gcccctcctc ctgctcctgc ccctcctgcc cctcctcctg ctcctgcccc tcctcctgct 5220
   cctgcccctc ctgcccctcc tgcccctcct cctgctcctg cccctcctgc ccctcctcct 5280
   gctcctgccc ctcctcctgc tcctgcccct cctgcccctc ctgcccctcc tcctgctcct 5340
   gcccctcctc ctgctcctgc ccctcctgcc cctcctgccc ctcctgcccc tcctcctgct 5400
   cctgcccctc ctcctgctcc tgcccctcct gctcctgccc ctcccgctcc tgctcctgct 5460
   cctgttccac cgtgggtccc tttgcagcca atgcaacttg gacgtttttg gggtctccgg 5520
   acaccatctc tatgtcttgg ccctgatcct gagccgcccg gggctcctgg tcttccgcct 5580
   cctcgtcctc gtcctcttcc ccgtcctcgt ccatggttat caccccctct tctttgaggt 5640
   ccactgccgc cggagccttc tggtccagat gtgtctccct tctctcctag gccatttcca 5700
   ggtcctgtac ctggcccctc gtcagacatg attcacacta aaagagatca atagacatct 5760
   ttattagacg acgctcagtg aatacaggga gtgcagactc ctgccccctc caacagcccc 5820
   cccaccctca tccccttcat ggtcgctgtc agacagatcc aggtctgaaa attccccatc 5880
   ctccgaacca tcctcgtcct catcaccaat tactcgcagc ccggaaaact cccgctgaac 5940
   atcctcaaga tttgcgtcct gagcctcaag ccaggcctca aattcctcgt cccccttttt 6000
   gctggacggt agggatgggg attctcggga cccctcctct tcctcttcaa ggtcaccaga 6060
   cagagatgct actggggcaa cggaagaaaa gctgggtgcg gcctgtgagg atcagcttat 6120
   cgatgataag ctgtcaaaca tgagaattct tgaagacgaa agggcctcgt gatacgccta 6180
   tttttatagg ttaatgtcat gataataatg gtttcttaga cgtcaggtgg cacttttcgg 6240
   ggaaatgtgc gcggaacccc tatttgttta tttttctaaa tacattcaaa tatgtatccg 6300
   ctcatgagac aataaccctg ataaatgctt caataatatt gaaaaaggaa gagtatgagt 6360
   attcaacatt tccgtgtcgc ccttattccc ttttttgcgg cattttgcct tcctgttttt 6420
   gctcacccag aaacgctggt gaaagtaaaa gatgctgaag atcagttggg tgcacgagtg 6480
   ggttacatcg aactggatct caacagcggt aagatccttg agagttttcg ccccgaagaa 6540
   cgttttccaa tgatgagcac ttttaaagtt ctgctatgtg gcgcggtatt atcccgtgtt 6600
   gacgccgggc aagagcaact cggtcgccgc atacactatt ctcagaatga cttggttgag 6660
   tactcaccag tcacagaaaa gcatcttacg gatggcatga cagtaagaga attatgcagt 6720
   gctgccataa ccatgagtga taacactgcg gccaacttac ttctgacaac gatcggagga 6780
   ccgaaggagc taaccgcttt tttgcacaac atgggggatc atgtaactcg ccttgatcgt 6840
   tgggaaccgg agctgaatga agccatacca aacgacgagc gtgacaccac gatgcctgca 6900
   gcaatggcaa caacgttgcg caaactatta actggcgaac tacttactct agcttcccgg 6960
   caacaattaa tagactggat ggaggcggat aaagttgcag gaccacttct gcgctcggcc 7020
   cttccggctg gctggtttat tgctgataaa tctggagccg gtgagcgtgg gtctcgcggt 7080
   atcattgcag cactggggcc agatggtaag ccctcccgta tcgtagttat ctacacgacg 7140
   gggagtcagg caactatgga tgaacgaaat agacagatcg ctgagatagg tgcctcactg 7200
   attaagcatt ggtaactgtc agaccaagtt tactcatata tactttagat tgatttaaaa 7260
   cttcattttt aatttaaaag gatctaggtg aagatccttt ttgataatct catgaccaaa 7320
   atcccttaac gtgagttttc gttccactga gcgtcagacc ccgtagaaaa gatcaaagga 7380
   tcttcttgag atcctttttt tctgcgcgta atctgctgct tgcaaacaaa aaaaccaccg 7440
   ctaccagcgg tggtttgttt gccggatcaa gagctaccaa ctctttttcc gaaggtaact 7500
   ggcttcagca gagcgcagat accaaatact gtccttctag tgtagccgta gttaggccac 7560
   cacttcaaga actctgtagc accgcctaca tacctcgctc tgctaatcct gttaccagtg 7620
   gctgctgcca gtggcgataa gtcgtgtctt accgggttgg actcaagacg atagttaccg 7680
   gataaggcgc agcggtcggg ctgaacgggg ggttcgtgca cacagcccag cttggagcga 7740
   acgacctaca ccgaactgag atacctacag cgtgagctat gagaaagcgc cacgcttccc 7800
   gaagggagaa aggcggacag gtatccggta agcggcaggg tcggaacagg agagcgcacg 7860
   agggagcttc cagggggaaa cgcctggtat ctttatagtc ctgtcgggtt tcgccacctc 7920
   tgacttgagc gtcgattttt gtgatgctcg tcaggggggc ggagcctatg gaaaaacgcc 7980
   agcaacgcgg cctttttacg gttcctggcc ttttgctggc cttgaagctg tccctgatgg 8040
   tcgtcatcta cctgcctgga cagcatggcc tgcaacgcgg gcatcccgat gccgccggaa 8100
   gcgagaagaa tcataatggg gaaggccatc cagcctcgcg tcgcgaacgc cagcaagacg 8160
   tagcccagcg cgtcggcccc gagatgcgcc gcgtgcggct gctggagatg gcggacgcga 8220
   tggatatgtt ctgccaaggg ttggtttgcg cattcacagt tctccgcaag aattgattgg 8280
   ctccaattct tggagtggtg aatccgttag cgaggtgccg ccctgcttca tccccgtggc 8340
   ccgttgctcg cgtttgctgg cggtgtcccc ggaagaaata tatttgcatg tctttagttc 8400
   tatgatgaca caaaccccgc ccagcgtctt gtcattggcg aattcgaaca cgcagatgca 8460
   gtcggggcgg cgcggtccga ggtccacttc gcatattaag gtgacgcgtg tggcctcgaa 8520
   caccgagcga ccctgcagcg acccgcttaa cagcgtcaac agcgtgccgc agatcccggg 8580
   gggcaatgag atatgaaaaa gcctgaactc accgcgacgt ctgtcgagaa gtttctgatc 8640
   gaaaagttcg acagcgtctc cgacctgatg cagctctcgg agggcgaaga atctcgtgct 8700
   ttcagcttcg atgtaggagg gcgtggatat gtcctgcggg taaatagctg cgccgatggt 8760
   ttctacaaag atcgttatgt ttatcggcac tttgcatcgg ccgcgctccc gattccggaa 8820
   gtgcttgaca ttggggaatt cagcgagagc ctgacctatt gcatctcccg ccgtgcacag 8880
   ggtgtcacgt tgcaagacct gcctgaaacc gaactgcccg ctgttctgca gccggtcgcg 8940
   gaggccatgg atgcgatcgc tgcggccgat cttagccaga cgagcgggtt cggcccattc 9000
   ggaccgcaag gaatcggtca atacactaca tggcgtgatt tcatatgcgc gattgctgat 9060
   ccccatgtgt atcactggca aactgtgatg gacgacaccg tcagtgcgtc cgtcgcgcag 9120
   gctctcgatg agctgatgct ttgggccgag gactgccccg aagtccggca cctcgtgcac 9180
   gcggatttcg gctccaacaa tgtcctgacg gacaatggcc gcataacagc ggtcattgac 9240
   tggagcgagg cgatgttcgg ggattcccaa tacgaggtcg ccaacatctt cttctggagg 9300
   ccgtggttgg cttgtatgga gcagcagacg cgctacttcg agcggaggca tccggagctt 9360
   gcaggatcgc cgcggctccg ggcgtatatg ctccgcattg gtcttgacca actctatcag 9420
   agcttggttg acggcaattt cgatgatgca gcttgggcgc agggtcgatg cgacgcaatc 9480
   gtccgatccg gagccgggac tgtcgggcgt acacaaatcg cccgcagaag cgcggccgtc 9540
   tggaccgatg gctgtgtaga agtactcgcc gatagtggaa accgacgccc cagcactcgt 9600
   ccggatcggg agatggggga ggctaactga aacacggaag gagacaatac cggaaggaac 9660
   ccgcgctatg acggcaataa aaagacagaa taaaacgcac gggtgttggg tcgtttgttc 9720
   ataaacgcgg ggttcggtcc cagggctggc actctgtcga taccccaccg agaccccatt 9780
   ggggccaata cgcccgcgtt tcttcctttt ccccacccca ccccccaagt tcgggtgaag 9840
   gcccagggct cgcagccaac gtcggggcgg caggccctgc catagccact ggccccgtgg 9900
   gttagggacg gggtccccca tggggaatgg tttatggttc gtgggggtta ttattttggg 9960
   cgttgcgtgg ggtcaggtcc acgactggac tgagcagaca gacccatggt ttttggatgg 10020
   cctgggcatg gaccgcatgt actggcgcga cacgaacacc gggcgtctgt ggctgccaaa 10080
   cacccccgac ccccaaaaac caccgcgcgg atttctggcg tgccaagcta gtcgaccaat 10140
   tctcatgttt gacagcttat catcgcagat ccgggcaacg ttgttgccat tgctgcaggc 10200
   gcagaactgg taggtatgga agatctatac attgaatcaa tattggcaat tagccatatt 10260
   agtcattggt tatatagcat aaatcaatat tggctattgg ccattgcata cgttgtatct 10320
   atatcataat atgtacattt atattggctc atgtccaata tgaccgccat gttgacattg 10380
   attattgact agttattaat agtaatcaat tacggggtca ttagttcata gcccatatat 10440
   ggagttccgc gttacataac ttacggtaaa tggcccgcct ggctgaccgc ccaacgaccc 10500
   ccgcccattg acgtcaataa tgacgtatgt tcccatagta acgccaatag ggactttcca 10560
   ttgacgtcaa tgggtggagt atttacggta aactgcccac ttggcagtac atcaagtgta 10620
   tcatatgcca agtccgcccc ctattgacgt caatgacggt aaatggcccg cctggcatta 10680
   tgcccagtac atgaccttac gggactttcc tacttggcag tacatctacg tattagtcat 10740
   cgctattacc atggtgatgc ggttttggca gtacaccaat gggcgtggat agcggtttga 10800
   ctcacgggga tttccaagtc tccaccccat tgacgtcaat gggagtttgt tttggcacca 10860
   aaatcaacgg gactttccaa aatgtcgtaa taaccccgcc ccgttgacgc aaatgggcgg 10920
   taggcgtgta cggtgggagg tctatataag cagagctcgt ttagtgaacc gtcagatctc 10980
   tagaagctgg gtac 10994

<210> 9
<211> 12539
<212> DNA
<213> artificial

<220>
<223> plasmid vector encoding Fc-tag IL-12 fusion construct

<400> 9
gtacctcgca gcaaagcaag atgtgtcctc agaagctaac catctcctgg tttgccatcg 60

ttttgctggt gtctccactc atggccatgt gggagctgga gaaagacgtt tatgttgtag 120

aggtggactg gactcccgat gcccctggag aaacagtgaa cctcacctgt gacacgcctg 180

aagaagatga catcacctgg acctcagacc agagacatgg agtcataggc tctggaaaga 240

ccctgaccat cactgtcaaa gagtttctag atgctggcca gtacacctgc cacaaaggag 300

gcgagactct gagccactca catctgctgc tccacaagaa ggaaaatgga atttggtcca 360

ctgaaatttt aaaaaatttc aaaaacaaga ctttcctgaa gtgtgaagca ccaaattact 420

ccggacggtt cacgtgctca tggctggtgc aaagaaacat ggacttgaag ttcaacatca 480

agagcagtag cagttcccct gactctcggg cagtgacatg tggaatggcg tctctgtctg 540

cagagaaggt cacactggac caaagggact atgagaagta ttcagtgtcc tgccaggagg 600

atgtcacctg cccaactgcc gaggagaccc tgcccattga actggcgttg gaagcacggc 660

agcagaataa atatgagaac tacagcacca gcttcttcat cagggacatc atcaaaccag 720

acccgcccaa gaacttgcag atgaagcctt tgaagaactc acaggtggag gtcagctggg 780

agtaccctga ctcctggagc actccccatt cctacttctc cctcaagttc tttgttcgaa 840

tccagcgcaa gaaagaaaag atgaaggaga cagaggaggg gtgtaaccag aaaggtgcgt 900

tcctcgtaga gaagacatct accgaagtcc aatgcaaagg cgggaatgtc tgcgtgcaag 960

ctcaggatcg ctattacaat tcctcgtgca gcaagtgggc atgtgttccc tgcagggtcc 1020

gatccggagg cggtggctcg ggcggtggtg ggtcgggtgg cggcggatcc agggtcattc 1080

cagtctctgg acctgccagg tgtcttagcc agtcccgaaa cctgctgaag accacagatg 1140

acatggtgaa gacggccaga gaaaaactga aacattattc ctgcactgct gaagacatcg 1200

atcatgaaga catcacacgg gaccaaacca gcacattgaa gacctgttta ccactggaac 1260

tacacaagaa cgagagttgc ctggctacta gagagacttc ttccacaaca agagggagct 1320

gcctgccccc acagaagacg tctttgatga tgaccctgtg ccttggtagc atctatgagg 1380

acttgaagat gtaccagaca gagttccagg ccatcaacgc agcacttcag aatcacaacc 1440

atcagcagat cattctagac aagggcatgc tggtggccat cgatgagctg atgcagtctc 1500

tgaatcataa tggcgagact ctgcgccaga aacctcctgt gggagaagca gacccttaca 1560

gagtgaaaat gaagctctgc atcctgcttc acgccttcag cacccgcgtc gtgaccatca 1620

acagggtgat gggctatctg agctccgcct tgatcaagag aatcgagcct agaataccca 1680

agcccagtac ccccccaggt tcttcatgcc cacctggtaa catcttgggt ggaccatccg 1740

tcttcatctt ccccccaaag cccaaggatg cactcatgat ctccctaacc cccaaggtta 1800

cgtgtgtggt ggtggatgtg agcgaggatg acccagatgt ccatgtcagc tggtttgtgg 1860

acaacaaaga agtacacaca gcctggacgc agccccgtga agctcagtac aacagtacct 1920

tccgagtggt cagtgccctc cccatccagc accaggactg gatgaggggc aaggagttca 1980

aatgcaaggt caacaacaaa gccctcccag cccccatcga gagaaccatc tcaaaaccca 2040

aaggaagagc ccagacacct caagtataca ccataccccc acctcgtgaa caaatgtcca 2100

agaagaaggt tagtctgacc tgcctggtca ccaacttctt ctctgaagcc atcagtgtgg 2160

agtgggaaag gaacggagaa ctggagcagg attacaagaa cactccaccc atcctggact 2220

cggatgggac ctacttcctc tacagcaagc tcactgtgga tacagacagt tggttgcaag 2280

gagaaatttt tacctgctcc gtggtgcatg aggctctcca taaccaccac acacagaaga 2340

acctgtctcg ctcccctggt aaatgagaac agcatctagc ggccgctcga ggccggcaag 2400

gccggatcca gacatgataa gatacattga tgagtttgga caaaccacaa ctagaatgca 2460

gtgaaaaaaa tgctttattt gtgaaatttg tgatgctatt gctttatttg taaccattat 2520

aagctgcaat aaacaagtta acaacaacaa ttgcattcat tttatgtttc aggttcaggg 2580

ggaggtgtgg gaggtttttt aaagcaagta aaacctctac aaatgtggta tggctgatta 2640

tgatccggct gcctcgcgcg tttcggtgat gacggtgaaa acctctgaca catgcagctc 2700

ccggagacgg tcacagcttg tctgtaagcg gatgccggga gcagacaagc ccgtcaggcg 2760

tcagcgggtg ttggcgggtg tcggggcgca gccatgaggt cgactctaga ggatcgatgc 2820

cccgccccgg acgaactaaa cctgactacg acatctctgc cccttcttcg cggggcagtg 2880

catgtaatcc cttcagttgg ttggtacaac ttgccaactg ggccctgttc cacatgtgac 2940

acgggggggg accaaacaca aaggggttct ctgactgtag ttgacatcct tataaatgga 3000

tgtgcacatt tgccaacact gagtggcttt catcctggag cagactttgc agtctgtgga 3060

ctgcaacaca acattgcctt tatgtgtaac tcttggctga agctcttaca ccaatgctgg 3120

gggacatgta cctcccaggg gcccaggaag actacgggag gctacaccaa cgtcaatcag 3180

aggggcctgt gtagctaccg ataagcggac cctcaagagg gcattagcaa tagtgtttat 3240

aaggccccct tgttaaccct aaacgggtag catatgcttc ccgggtagta gtatatacta 3300

tccagactaa ccctaattca atagcatatg ttacccaacg ggaagcatat gctatcgaat 3360

tagggttagt aaaagggtcc taaggaacag cgatatctcc caccccatga gctgtcacgg 3420

ttttatttac atggggtcag gattccacga gggtagtgaa ccattttagt cacaagggca 3480

gtggctgaag atcaaggagc gggcagtgaa ctctcctgaa tcttcgcctg cttcttcatt 3540

ctccttcgtt tagctaatag aataactgct gagttgtgaa cagtaaggtg tatgtgaggt 3600

gctcgaaaac aaggtttcag gtgacgcccc cagaataaaa tttggacggg gggttcagtg 3660

gtggcattgt gctatgacac caatataacc ctcacaaacc ccttgggcaa taaatactag 3720

tgtaggaatg aaacattctg aatatcttta acaatagaaa tccatggggt ggggacaagc 3780

cgtaaagact ggatgtccat ctcacacgaa tttatggcta tgggcaacac ataatcctag 3840

tgcaatatga tactggggtt attaagatgt gtcccaggca gggaccaaga caggtgaacc 3900

atgttgttac actctatttg taacaagggg aaagagagtg gacgccgaca gcagcggact 3960

ccactggttg tctctaacac ccccgaaaat taaacggggc tccacgccaa tggggcccat 4020

aaacaaagac aagtggccac tctttttttt gaaattgtgg agtgggggca cgcgtcagcc 4080

cccacacgcc gccctgcggt tttggactgt aaaataaggg tgtaataact tggctgattg 4140

taaccccgct aaccactgcg gtcaaaccac ttgcccacaa aaccactaat ggcaccccgg 4200

ggaatacctg cataagtagg tgggcgggcc aagatagggg cgcgattgct gcgatctgga 4260

ggacaaatta cacacacttg cgcctgagcg ccaagcacag ggttgttggt cctcatattc 4320

acgaggtcgc tgagagcacg gtgggctaat gttgccatgg gtagcatata ctacccaaat 4380

atctggatag catatgctat cctaatctat atctgggtag cataggctat cctaatctat 4440

atctgggtag catatgctat cctaatctat atctgggtag tatatgctat cctaatttat 4500

atctgggtag cataggctat cctaatctat atctgggtag catatgctat cctaatctat 4560

atctgggtag tatatgctat cctaatctgt atccgggtag catatgctat cctaatagag 4620

attagggtag tatatgctat cctaatttat atctgggtag catatactac ccaaatatct 4680

ggatagcata tgctatccta atctatatct gggtagcata tgctatccta atctatatct 4740

gggtagcata ggctatccta atctatatct gggtagcata tgctatccta atctatatct 4800

gggtagtata tgctatccta atttatatct gggtagcata ggctatccta atctatatct 4860

gggtagcata tgctatccta atctatatct gggtagtata tgctatccta atctgtatcc 4920

gggtagcata tgctatcctc atgcatatac agtcagcata tgatacccag tagtagagtg 4980

ggagtgctat cctttgcata tgccgccacc tcccaagggg gcgtgaattt tcgctgcttg 5040

tccttttcct gctggttgct cccattctta ggtgaattta aggaggccag gctaaagccg 5100

tcgcatgtct gattgctcac caggtaaatg tcgctaatgt tttccaacgc gagaaggtgt 5160

tgagcgcgga gctgagtgac gtgacaacat gggtatgccc aattgcccca tgttgggagg 5220

acgaaaatgg tgacaagaca gatggccaga aatacaccaa cagcacgcat gatgtctact 5280

ggggatttat tctttagtgc gggggaatac acggctttta atacgattga gggcgtctcc 5340

taacaagtta catcactcct gcccttcctc accctcatct ccatcacctc cttcatctcc 5400

gtcatctccg tcatcaccct ccgcggcagc cccttccacc ataggtggaa accagggagg 5460

caaatctact ccatcgtcaa agctgcacac agtcaccctg atattgcagg taggagcggg 5520

ctttgtcata acaaggtcct taatcgcatc cttcaaaacc tcagcaaata tatgagtttg 5580

taaaaagacc atgaaataac agacaatgga ctcccttagc gggccaggtt gtgggccggg 5640

tccaggggcc attccaaagg ggagacgact caatggtgta agacgacatt gtggaatagc 5700

aagggcagtt cctcgcctta ggttgtaaag ggaggtctta ctacctccat atacgaacac 5760

accggcgacc caagttcctt cgtcggtagt cctttctacg tgactcctag ccaggagagc 5820

tcttaaacct tctgcaatgt tctcaaattt cgggttggaa cctccttgac cacgatgctt 5880

tccaaaccac cctccttttt tgcgcctgcc tccatcaccc tgaccccggg gtccagtgct 5940

tgggccttct cctgggtcat ctgcggggcc ctgctctatc gctcccgggg gcacgtcagg 6000

ctcaccatct gggccacctt cttggtggta ttcaaaataa tcggcttccc ctacagggtg 6060

gaaaaatggc cttctacctg gaggggggct gcgcggtgga gacccggatg atgatgactg 6120

actactggga ctcctgggcc tcttttctcc acgtccacga cctctccccc tggctctttc 6180

acgacttccc cccctggctc tttcacgtcc tctaccccgg cggcctccac tacctcctcg 6240

accccggcct ccactacctc ctcgaccccg gcctccactg cctcctcgac cccggcctcc 6300

acctcctgct cctgcccctc ctgctcctgc ccctcctcct gctcctgccc ctcctgcccc 6360

tcctgctcct gcccctcctg cccctcctgc tcctgcccct cctgcccctc ctgctcctgc 6420

ccctcctgcc cctcctcctg ctcctgcccc tcctgcccct cctcctgctc ctgcccctcc 6480

tgcccctcct gctcctgccc ctcctgcccc tcctgctcct gcccctcctg cccctcctgc 6540

tcctgcccct cctgctcctg cccctcctgc tcctgcccct cctgctcctg cccctcctgc 6600

ccctcctgcc cctcctcctg ctcctgcccc tcctgctcct gcccctcctg cccctcctgc 6660

ccctcctgct cctgcccctc ctcctgctcc tgcccctcct gcccctcctg cccctcctcc 6720

tgctcctgcc cctcctgccc ctcctcctgc tcctgcccct cctcctgctc ctgcccctcc 6780

tgcccctcct gcccctcctc ctgctcctgc ccctcctgcc cctcctcctg ctcctgcccc 6840

tcctcctgct cctgcccctc ctgcccctcc tgcccctcct cctgctcctg cccctcctcc 6900

tgctcctgcc cctcctgccc ctcctgcccc tcctgcccct cctcctgctc ctgcccctcc 6960

tcctgctcct gcccctcctg ctcctgcccc tcccgctcct gctcctgctc ctgttccacc 7020

gtgggtccct ttgcagccaa tgcaacttgg acgtttttgg ggtctccgga caccatctct 7080

atgtcttggc cctgatcctg agccgcccgg ggctcctggt cttccgcctc ctcgtcctcg 7140

tcctcttccc cgtcctcgtc catggttatc accccctctt ctttgaggtc cactgccgcc 7200

ggagccttct ggtccagatg tgtctccctt ctctcctagg ccatttccag gtcctgtacc 7260

tggcccctcg tcagacatga ttcacactaa aagagatcaa tagacatctt tattagacga 7320

cgctcagtga atacagggag tgcagactcc tgccccctcc aacagccccc ccaccctcat 7380

ccccttcatg gtcgctgtca gacagatcca ggtctgaaaa ttccccatcc tccgaaccat 7440

cctcgtcctc atcaccaatt actcgcagcc cggaaaactc ccgctgaaca tcctcaagat 7500

ttgcgtcctg agcctcaagc caggcctcaa attcctcgtc cccctttttg ctggacggta 7560

gggatgggga ttctcgggac ccctcctctt cctcttcaag gtcaccagac agagatgcta 7620

ctggggcaac ggaagaaaag ctgggtgcgg cctgtgagga tcagcttatc gatgataagc 7680

tgtcaaacat gagaattctt gaagacgaaa gggcctcgtg atacgcctat ttttataggt 7740

taatgtcatg ataataatgg tttcttagac gtcaggtggc acttttcggg gaaatgtgcg 7800

cggaacccct atttgtttat ttttctaaat acattcaaat atgtatccgc tcatgagaca 7860

ataaccctga taaatgcttc aataatattg aaaaaggaag agtatgagta ttcaacattt 7920

ccgtgtcgcc cttattccct tttttgcggc attttgcctt cctgtttttg ctcacccaga 7980

aacgctggtg aaagtaaaag atgctgaaga tcagttgggt gcacgagtgg gttacatcga 8040

actggatctc aacagcggta agatccttga gagttttcgc cccgaagaac gttttccaat 8100

gatgagcact tttaaagttc tgctatgtgg cgcggtatta tcccgtgttg acgccgggca 8160

agagcaactc ggtcgccgca tacactattc tcagaatgac ttggttgagt actcaccagt 8220

cacagaaaag catcttacgg atggcatgac agtaagagaa ttatgcagtg ctgccataac 8280

catgagtgat aacactgcgg ccaacttact tctgacaacg atcggaggac cgaaggagct 8340

aaccgctttt ttgcacaaca tgggggatca tgtaactcgc cttgatcgtt gggaaccgga 8400

gctgaatgaa gccataccaa acgacgagcg tgacaccacg atgcctgcag caatggcaac 8460

aacgttgcgc aaactattaa ctggcgaact acttactcta gcttcccggc aacaattaat 8520

agactggatg gaggcggata aagttgcagg accacttctg cgctcggccc ttccggctgg 8580

ctggtttatt gctgataaat ctggagccgg tgagcgtggg tctcgcggta tcattgcagc 8640

actggggcca gatggtaagc cctcccgtat cgtagttatc tacacgacgg ggagtcaggc 8700

aactatggat gaacgaaata gacagatcgc tgagataggt gcctcactga ttaagcattg 8760

gtaactgtca gaccaagttt actcatatat actttagatt gatttaaaac ttcattttta 8820

atttaaaagg atctaggtga agatcctttt tgataatctc atgaccaaaa tcccttaacg 8880

tgagttttcg ttccactgag cgtcagaccc cgtagaaaag atcaaaggat cttcttgaga 8940

tccttttttt ctgcgcgtaa tctgctgctt gcaaacaaaa aaaccaccgc taccagcggt 9000

ggtttgtttg ccggatcaag agctaccaac tctttttccg aaggtaactg gcttcagcag 9060

agcgcagata ccaaatactg tccttctagt gtagccgtag ttaggccacc acttcaagaa 9120

ctctgtagca ccgcctacat acctcgctct gctaatcctg ttaccagtgg ctgctgccag 9180

tggcgataag tcgtgtctta ccgggttgga ctcaagacga tagttaccgg ataaggcgca 9240

gcggtcgggc tgaacggggg gttcgtgcac acagcccagc ttggagcgaa cgacctacac 9300

cgaactgaga tacctacagc gtgagctatg agaaagcgcc acgcttcccg aagggagaaa 9360

ggcggacagg tatccggtaa gcggcagggt cggaacagga gagcgcacga gggagcttcc 9420

agggggaaac gcctggtatc tttatagtcc tgtcgggttt cgccacctct gacttgagcg 9480

tcgatttttg tgatgctcgt caggggggcg gagcctatgg aaaaacgcca gcaacgcggc 9540

ctttttacgg ttcctggcct tttgctggcc ttgaagctgt ccctgatggt cgtcatctac 9600

ctgcctggac agcatggcct gcaacgcggg catcccgatg ccgccggaag cgagaagaat 9660

cataatgggg aaggccatcc agcctcgcgt cgcgaacgcc agcaagacgt agcccagcgc 9720

gtcggccccg agatgcgccg cgtgcggctg ctggagatgg cggacgcgat ggatatgttc 9780

tgccaagggt tggtttgcgc attcacagtt ctccgcaaga attgattggc tccaattctt 9840

ggagtggtga atccgttagc gaggtgccgc cctgcttcat ccccgtggcc cgttgctcgc 9900

gtttgctggc ggtgtccccg gaagaaatat atttgcatgt ctttagttct atgatgacac 9960

aaaccccgcc cagcgtcttg tcattggcga attcgaacac gcagatgcag tcggggcggc 10020

gcggtccgag gtccacttcg catattaagg tgacgcgtgt ggcctcgaac accgagcgac 10080

cctgcagcga cccgcttaac agcgtcaaca gcgtgccgca gatcccgggg ggcaatgaga 10140

tatgaaaaag cctgaactca ccgcgacgtc tgtcgagaag tttctgatcg aaaagttcga 10200

cagcgtctcc gacctgatgc agctctcgga gggcgaagaa tctcgtgctt tcagcttcga 10260

tgtaggaggg cgtggatatg tcctgcgggt aaatagctgc gccgatggtt tctacaaaga 10320

tcgttatgtt tatcggcact ttgcatcggc cgcgctcccg attccggaag tgcttgacat 10380

tggggaattc agcgagagcc tgacctattg catctcccgc cgtgcacagg gtgtcacgtt 10440

gcaagacctg cctgaaaccg aactgcccgc tgttctgcag ccggtcgcgg aggccatgga 10500

tgcgatcgct gcggccgatc ttagccagac gagcgggttc ggcccattcg gaccgcaagg 10560

aatcggtcaa tacactacat ggcgtgattt catatgcgcg attgctgatc cccatgtgta 10620

tcactggcaa actgtgatgg acgacaccgt cagtgcgtcc gtcgcgcagg ctctcgatga 10680

gctgatgctt tgggccgagg actgccccga agtccggcac ctcgtgcacg cggatttcgg 10740

ctccaacaat gtcctgacgg acaatggccg cataacagcg gtcattgact ggagcgaggc 10800

gatgttcggg gattcccaat acgaggtcgc caacatcttc ttctggaggc cgtggttggc 10860

ttgtatggag cagcagacgc gctacttcga gcggaggcat ccggagcttg caggatcgcc 10920

gcggctccgg gcgtatatgc tccgcattgg tcttgaccaa ctctatcaga gcttggttga 10980

cggcaatttc gatgatgcag cttgggcgca gggtcgatgc gacgcaatcg tccgatccgg 11040

agccgggact gtcgggcgta cacaaatcgc ccgcagaagc gcggccgtct ggaccgatgg 11100

ctgtgtagaa gtactcgccg atagtggaaa ccgacgcccc agcactcgtc cggatcggga 11160

gatgggggag gctaactgaa acacggaagg agacaatacc ggaaggaacc cgcgctatga 11220

cggcaataaa aagacagaat aaaacgcacg ggtgttgggt cgtttgttca taaacgcggg 11280

gttcggtccc agggctggca ctctgtcgat accccaccga gaccccattg gggccaatac 11340

gcccgcgttt cttccttttc cccaccccac cccccaagtt cgggtgaagg cccagggctc 11400

gcagccaacg tcggggcggc aggccctgcc atagccactg gccccgtggg ttagggacgg 11460

ggtcccccat ggggaatggt ttatggttcg tgggggttat tattttgggc gttgcgtggg 11520

gtcaggtcca cgactggact gagcagacag acccatggtt tttggatggc ctgggcatgg 11580

accgcatgta ctggcgcgac acgaacaccg ggcgtctgtg gctgccaaac acccccgacc 11640

cccaaaaacc accgcgcgga tttctggcgt gccaagctag tcgaccaatt ctcatgtttg 11700

acagcttatc atcgcagatc cgggcaacgt tgttgccatt gctgcaggcg cagaactggt 11760

aggtatggaa gatctataca ttgaatcaat attggcaatt agccatatta gtcattggtt 11820

atatagcata aatcaatatt ggctattggc cattgcatac gttgtatcta tatcataata 11880

tgtacattta tattggctca tgtccaatat gaccgccatg ttgacattga ttattgacta 11940

gttattaata gtaatcaatt acggggtcat tagttcatag cccatatatg gagttccgcg 12000

ttacataact tacggtaaat ggcccgcctg gctgaccgcc caacgacccc cgcccattga 12060

cgtcaataat gacgtatgtt cccatagtaa cgccaatagg gactttccat tgacgtcaat 12120

gggtggagta tttacggtaa actgcccact tggcagtaca tcaagtgtat catatgccaa 12180

gtccgccccc tattgacgtc aatgacggta aatggcccgc ctggcattat gcccagtaca 12240

tgaccttacg ggactttcct acttggcagt acatctacgt attagtcatc gctattacca 12300

tggtgatgcg gttttggcag tacaccaatg ggcgtggata gcggtttgac tcacggggat 12360

ttccaagtct ccaccccatt gacgtcaatg ggagtttgtt ttggcaccaa aatcaacggg 12420

actttccaaa atgtcgtaat aaccccgccc cgttgacgca aatgggcggt aggcgtgtac 12480

ggtgggaggt ctatataagc agagctcgtt tagtgaaccg tcagatctct agaagctgg 12539

## Description

The present invention relates to compositions and methods for treating cancer, in particular, to immunotherapy of malignant neoplastic disease such as glioma, by administering an effective dose of a polypeptide with IL-12 biological activity and a non-agonist ligand to CTLA-4.

Glioblastoma multiforme (GBM) is the most malignant astrocytic tumor. GBM exhibits an invasive and destructive growth pattern; it is the most common and most aggressive malignant primary brain tumor in humans, accounting for 20 % of all intracranial tumors. In most European countries and North America, GBM incidence is in the range of 2-3 new cases per 100'000 population per year. The clinical history of the disease is usually short (less than 3 months in more than 50% of cases) and patients diagnosed with GBM show a median survival of 14 months despite aggressive surgery, radiation, and chemotherapy. The ability of gliomas to withstand conventional treatment regimens is one of the greatest challenges of modern neuro-oncology.

Interleukin (IL)-12 is the prototype of a group of heterodimeric cytokines with predominantly inflammatory properties. IL-12 polarizes naive helper T-cells to adopt a TH1 phenotype and stimulates cytotoxic T and NK-cells. IL-12 binds to the IL-12 receptor (IL-12R), which is a heterodimeric receptor formed by IL-12R-β1 and IL-12R-β2.The receptor complex is primarily expressed by T cells, but also other lymphocyte subpopulations have been found to be responsive to IL-12.

While research in recent years has mainly focused on various administration routes of IL-12, there remain open questions on the exact mechanisms by which IL-12 exerts its tumor-suppressive properties. The therapeutic application of IL-12 in various tumor entities has been suggested. Clinical trials in cancer patients, however, had to be halted since systemic application evoked serious adverse events at effective doses, including fatalities.While research in recent years has mainly focused on various administration routes of IL-12, there remain open questions on the exact mechanisms by which IL-12 exerts its tumor-suppressive properties.

CTLA-4 is expressed on the surface of T helper cells and transmits an inhibitory signal to T lymphocytes. CTLA-4 and CD28 bind to CD80 (B7-1) and CD86 (B7-2) on antigen-presenting cells. CTLA-4 transmits an inhibitory signal to T cells, whereas CD28 transmits a stimulatory signal. Systemic anti-CTLA-4 treatment has been approved for clinical use and demonstrates clinical benefit. It is being further tested for various other solid cancers (Hodi et al., N Engl J Med 363, 711-723 (2010); Graziani et al., Ipilimumab: A novel immunostimulatory monoclonal antibody for the treatment of cancer. Pharmacol Res (2011)). A commercial antibody against CTLA-4 is available under the generic name ipilimumab (marketed as *Yervoy*)*.*

The glycoprotein immunoglobulin G (IgG) is a major effector molecule of the humoral immune response in man. There are four distinct subgroups of human IgG designated IgG1, IgG2, IgG3 and IgG4. The four subclasses show more than 95% homology in the amino acid sequences of the constant domains of the heavy chains, but differ with respect to structure and flexibility of the hinge region, especially in the number of inter-heavy chain disulfide bonds in this domain. The structural differences between the IgG subclasses are also reflected in their susceptibility to proteolytic enzymes, such as papain, plasmin, trypsin and pepsin.

Only one isoform of human IgG4 is known. In contrast to human IgG1, IgG2 and IgG3, human IgG4 does not activate complement. Furthermore, IgG4 is less susceptible to proteolytic enzymes compared to IgG2 and IgG3.

The problem underlying the present invention is the provision of improved means and methods for treating solid cancer, in particular glioma.

In the course of a study focused on the clinical therapeutic potential of IL-12 in advanced-stage GBM in a relevant rodent model, it was surprisingly found that the combination of IL-12 with a blockade of co-inhibitory signals with anti-CTLA-4 antibody leads to almost complete tumor eradication and cure even at advanced disease stages.

According to a first aspect of the invention, a combination medicament is provided, which comprises an IL-12 polypeptide and a non-agonist CTLA-4 ligand.

In the context of the present invention, an IL-12 polypeptide is a polypeptide having an amino acid sequence comprising the sequence of p35 (Uniprot ID 29459, SEQ ID 05) or a functional homologue thereof, and comprising the sequence of p40 (Uniprot ID29460, SEQ ID 06) or a functional homologue thereof. In one embodiment, the IL-12 polypeptide has an amino acid sequence comprising both p35 and p40 sequences or homologues thereof as part of the same continuous amino acid chain. In another embodiment, the IL-12 polypeptide comprises two distinct amino acid chains, one comprising the p35 sequence and another one comprising the p40 sequence. The terminology "IL-12 polypeptide does not preclude the presence of non-IL-12 sequences, for example immunoglobulin sequences and fragments thereof, fused to the IL-12 sequences described herein.

The IL-12 polypeptide has a biological activity of IL-12. A biological activity of IL-12 in the context of the present invention is the stimulation of NK or T cells by said IL-12 polypeptide, most prominently the stimulation of T effector cells acting through perforin. According to one embodiment of the combination medicament, said IL-12 polypeptide comprises a polypeptide sequence at least 95%, 96%, 97%, 98% or 99% identical to the sequence of human p35 (SEQ ID 05), and a polypeptide sequence at least 95%, 96%, 97%, 98% or 99% identical to the sequence of human p40 (SEQ ID 06).

Identity in the context of the present invention is a single quantitative parameter representing the result of a sequence comparison position by position. Methods of sequence comparison are known in the art; the BLAST algorithm available publicly is an example.

According to one embodiment of the combination medicament, said IL-12 polypeptide is a recombinant human IL-12. According to one embodiment, said IL-12 polypeptide is a synthetic human IL-12. According to one embodiment, said IL-12 polypeptide is a fusion peptide comprising the crystallisable fragment (Fc region) of a human immunoglobulin. According to one embodiment, the IL-12 polypeptide comprises a crystallisable fragment of human immunoglobulin G (the second and third constant domain of the IgG molecule). According to one embodiment, the IL-12 polypeptide comprises a crystallisable fragment of human immunoglobulin G4. According to one embodiment, the IL-12 polypeptide has or comprises the sequence of SEQ ID 01. According to another embodiment, the IL-12 polypeptide comprises a sequence at least 95%, 96%, 97%, 98% or 99% identical to the sequence of SEQ ID 01.

Embodiments wherein IL-12 polypeptide chains are fused to immunoglobulin Fc fragments may show different pharmacokinetic behavior in comparison to the recombinant cytokine, which for some applications may confer a benefit.

In the context of the present invention, a non-agonist CTLA-4 ligand is a molecule that binds selectively to CTLA-4 under conditions prevailing in peripheral blood, without triggering the biological effect of CTLA-4 interaction with any of the physiological ligands of CTLA-4, namely CD80 and/or CD86. Particularly, a non-agonist CTLA-4 ligand does not lead to attenuated T cell activity when binding to CTLA-4 on the surface on a T-cell. In certain embodiments, the term "non-agonist CTLA-4 ligand" covers both antagonists of CTLA-4 and ligands that are neutral vis-à-vis CTLA-4 signaling. In certain preferred embodiments, non-agonist CTLA-4 ligands used in the present invention are able, when bound to CTLA-4, to sterically block interaction of CTLA-4 with its binding partners CD80 and/or CD86.

According to one embodiment, said non-agonist CTLA-4 ligand is a gamma immunoglobulin binding to CTLA-4, without triggering the physiological response of CTLA-4 interaction with its binding partners CD80 and/or CD86. Non-limiting examples for a CTLA-4 ligand are the clinically approved antibodies tremelimumab (CAS 745013-59-6) and ipilimumab (CAS No. 477202-00-9; Yervoy). The term "gamma immunoglobulin" in this context is intended to encompass both complete immunoglobulin molecules and functional fragments thereof, wherein the function is binding to CTLA-4 as laid out above.

According to one embodiment, the combination therapy comprises two distinct dosage forms, wherein said IL-12 polypeptide is provided as a dosage form for intratumoral injection or local injection in the vicinity of the tumor, and said non-agonist CTLA-4 ligand is provided as a dosage form for systemic delivery, particularly by intravenous injection. According to another embodiment, the IL-12 polypeptide is applied directly to the tumor draining lymph node.

According to another embodiment, the combination therapy comprises a dosage form whereby said IL-12 polypeptide is provided for intracranial injection.

According to another aspect of the invention, a combination medicament is provided as set forth above, for therapy of a malignant neoplastic disease. According to one embodiment, the malignant neoplastic disease is glioma. According to another embodiment, the disease is glioblastoma multiforme. According to yet another embodiment, the malignant neoplastic disease is meningioma. According to yet another embodiment, the malignant neoplastic disease is melanoma. According to yet another embodiment, the malignant neoplastic disease is pancreatic cancer. According to yet another embodiment, the malignant neoplastic disease is lung cancer. According to yet another embodiment, the malignant neoplastic disease is prostate cancer. According to yet another embodiment, the malignant neoplastic disease is bladder cancer.

According to one embodiment, the combination medicament comprises an IL-12 polypeptide having a biological activity of IL-12 provided as a fusion protein comprising the amino acid of human p40, the amino acid sequence of human p35 and the crystallisable fragment of human IgG4, said IL-12 polypeptide being formulated as a dosage form for intratumoural delivery. According to this embodiment, the combination medicament further comprises an immunoglobulin G raised against CTLA-4 as a non-agonist CTLA-4 ligand formulated as a dosage form for systemic delivery. According to this embodiment, the combination medicament is provided for the treatment of malignant neoplastic disease, particularly for glioma, glioblastoma multiforme, meningioma, melanoma, pancreatic cancer, lung cancer,prostate cancer or bladder cancer. According to yet another aspect of the invention, an IL-12 polypeptide having a biological activity of IL-12, and a non-agonist CTLA-4 ligand are used in the manufacture of a combination medicament for therapy of a malignant neoplastic disease, particularly of glioma and other solid tissue tumors, such as glioblastoma multiforme, meningioma, melanoma, pancreatic cancer, lung cancer, prostate cancer or bladder cancer. According to yet another aspect of the invention, a method is provided for treating a patient suffering from malignant neoplastic disease, particularly glioma and other solid tissue tumors, comprising the administration of an IL-12 polypeptide having a biological activity of IL-12, and a non-agonist CTLA-4 ligand to said patient.

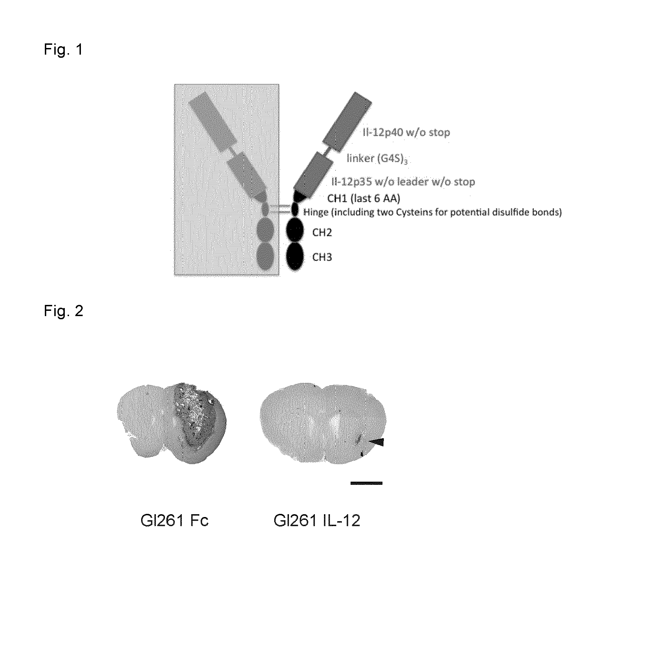

According to yet another aspect of the invention, a combination therapy comprises an IL-12 nucleic acid expression vector encoding an encoded IL-12 polypeptide having a biological activity of IL-12, and a protein non-agonist CTLA-4 ligand as set forth above. The encoded IL-12 polypeptide may have any of the characteristics set forth above. One non-limiting example for an encoded IL-12 polypeptide is a crystallisable immunoglobulin G fragment fused to the IL-12 constituent polypeptide chains, human IL-12 or a functional equivalent thereof. One non-limiting example is a fusion construct having the constituent polypeptides of IL-12 linked by a short amino acid sequence as depicted in Fig. 1, the amino acid sequence for which is given as SEQ ID 01 and the encoding nucleic acid sequence is given as SEQ ID 07.

The advantage of using fusion proteins cytokines and the crystallisable fragment of immunoglobulins rather than the recombinant cytokine is improved pharmacokinetics (Belladonna et al. J Immunol 168, 5448-5454 (2002); Schmidt, Curr Opin Drug Discov Devel 12, 284-295 (2009); Eisenring et al., Nat Immunol 11, 1030-1038 (2010)).

The IL-12 nucleic acid expression vector according to this aspect of the invention may, by way of non-limiting example, be a "naked" DNA expression plasmid comprising a nucleic acid sequence encoding the IL-12 polypeptide under control of a promoter sequence operable in a human tumor cell, for delivery into the tumor, for example by intracranial injection. The IL-12 nucleic acid expression vector may similarly be a viral vector, for example an adeno-associated virus, an adenovirus, a lentivirus or a herpes virus.

Such IL-12 nucleic acid expression vector may be provided for intratumoral delivery in combination with a protein non-agonist CTLA-4 ligand as set forth above. Similarly, the scope of the present invention encompasses the use of such IL-12 nucleic acid expression vector, in combination with a non-agonist CTLA-4 ligand, in a method of making a combination medicament for therapy of malignant neoplastic disease, particularly glioma, glioblastoma multiforme, meningioma, melanoma, pancreatic cancer, lung cancer, prostate cancer or bladder cancer. Likewise, a method is provided for treating a patient suffering from malignant neoplastic disease, particularly glioma or other solid tissue tumors, comprising the administration of an IL-12 nucleic acid expression vector having a biological activity of IL-12, anda non-agonist CTLA-4 ligand to said patient.

### Brief description of the Figures

- Fig. 1: shows the structure and sequence of the fusion protein given in SEQ ID 02.The subunits p40 and p35 of IL-12 are depicted as rectangles. These subunits are connected by a linker (G₄S)₃. The subunits CH2, CH3 and the last six amino acids of CH1 of the crystallizable fragment of the immunoglobulin are shown as oblong circles.
- Fig. 2: shows immunohistochemistry of formalin fixed tumor sections obtained from syngeneic C57/BI6 mice 5 weeks after challenge with 2x10⁴ GI261 IL-12Fc or GI261 Fc cells and stained with antibody against F4/80, counterstain hematoxylin (representative examples, n=6 mice per group). Scale bar indicates 2 mm, arrowhead indicates residual GI261 IL-12Fc tumor.

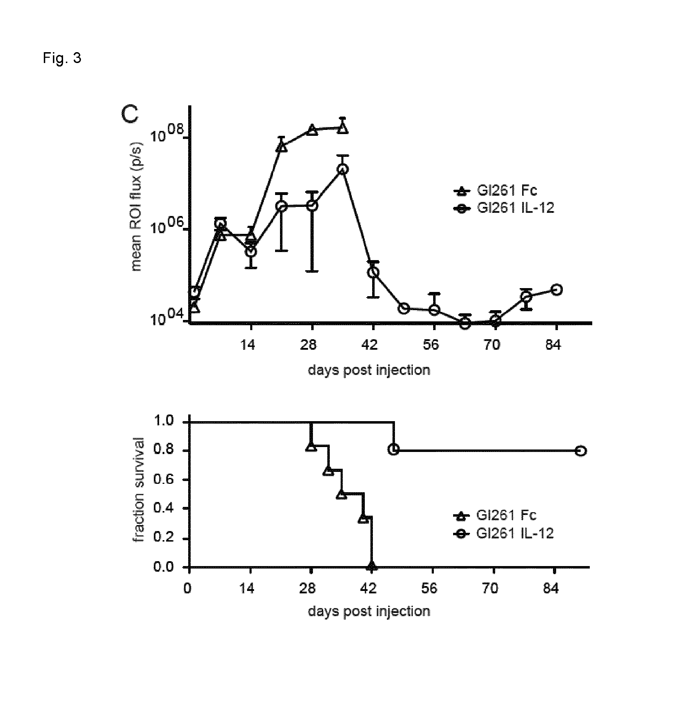

- Fig. 3: shows non-invasive Bioluminescence imaging (BLI) of mouse glioma in syngenic C57/BI6 mice (n = 5-6 mice per group) after implantation of 2x10⁴ GI261 cells constitutively expressing *photinuspyralis* luciferase and releasing a fusion protein of IL-12 and the crystallizable fragment of mouse immunoglobulin G3 (G1261 IL-12Fc) or Fc alone as control (G1261 Fc). Upper panel: Quantification of tumor growth which correlates to photon flux (p/s) in the region of interest (ROI) versus the days post injection of the modified glioma cells. Lower panel: Kaplan-Meier survival analysis. Data are representative of 2 independent experiments.

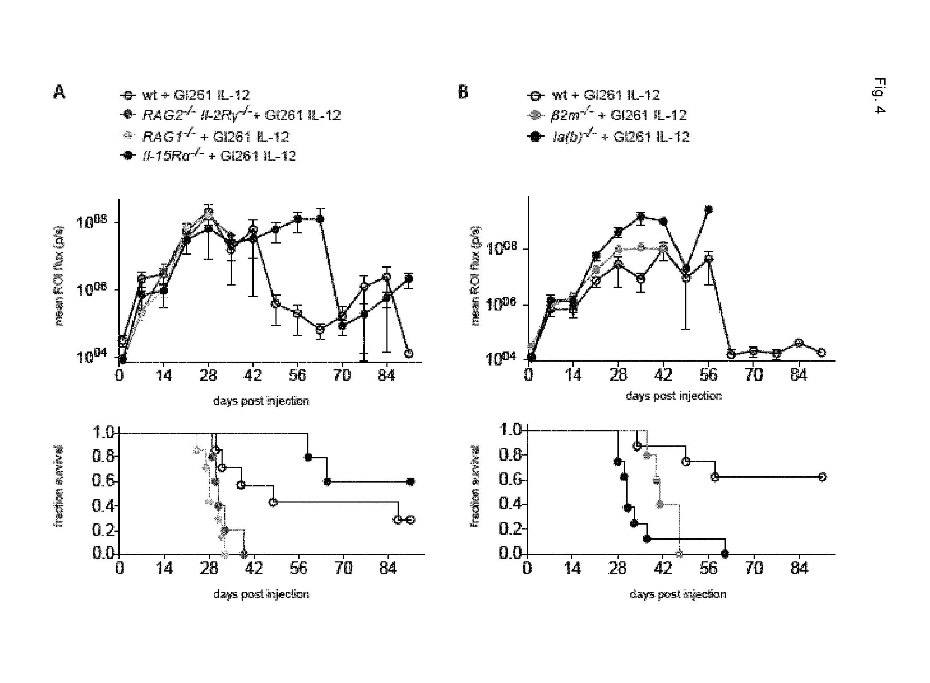

- Fig. 4: shows non-invasive Bioluminescence (BLI) imaging of mouse glioma in different mouse mutants (n = 5-7mice per group) after implantation of 2x10⁴ GI261 cells constitutively expressing *photinuspyralis* luciferase and releasing a fusion protein of IL-12 and the crystallizable fragment of mouse immunoglobulin G3 (G1261 IL-12Fc). The animals were challenged as described in Fig..Upper panel: Quantification of tumor growth versus the days post injection of the modified glioma cells. Lower panel: Kaplan-Meier survival analysis. A)GI261 IL-12Fc was implanted in mice lacking T and B cells (Rag1^{-/-}) or NK cells *(II-15ra*^{*-*/}*⁻)* or lacking both T-, B-, NK cells and lymphoid tissue inducer like cells *(Rag2*^{*-*/-}*II2rg*^{*-*/}*⁻).* B) Same as A); MHCII *(Ia(b)*^{*-*/}*⁻)* and MHCI (*β*2m^{-/-}) deficient mice.(n=5-8 mice/group). Data are representative of 2 independent experiments.

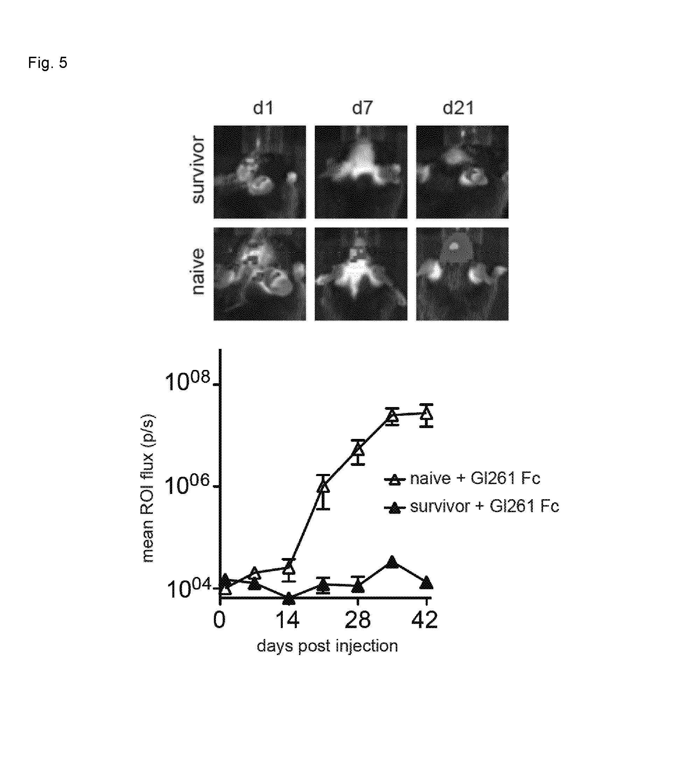

- Fig. 5: shows T cell memory formation in surviving wt animals that had been previously challenged with 2x10⁴ GI261 IL-12Fc cells. Examples for bioluminescence emitted from the brains of surviving wt animals that had been rechallenged with GI261 Fc cells compared to naïve wt animals is shown (upper panel, days 1,7 and 21 post rechallenge shown). Furthermore, bioluminescence of the tumors is shown in photons per second (p/s) in the region of interest (ROI) versus the days post injection of the modified glioma cells (lower panel). A rapid rejection of the control tumors in surviving wt animals was observed. While the measured luminescence at day 1 suggested identical seeding across the two groups, only the naïve mice exhibited a measurable signal at day 7 onwards, suggesting a rapid and effectively clearing anti-glioma memory response now independent of ectopically expressed pro-inflammatory cytokines (namely IL-12Fc).(n=4-6 mice/group). Data are representative of 2 independent experiments.

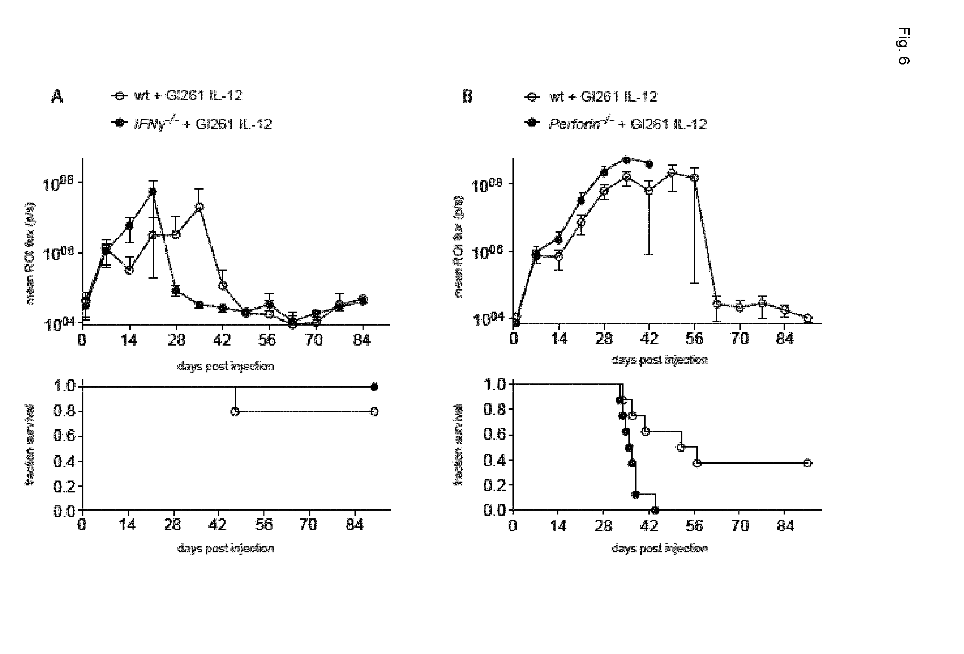

- Fig. 6: shows non-invasive Bioluminescence (BLI) imaging of mouse glioma in different mouse mutants (n = 4-8 mice per group) after implantation of 2x10⁴ GI261 Fccells constitutively expressing *photinuspyralis* luciferase and releasing a fusion protein of IL-12 and the crystallizable fragment of mouse immunoglobulin G3 (G1261 IL-12Fc). A) wt (open circles) and *IFNy*^{*-*/*-*} (black circles) animals B) wt (open circles) and *Perforin^{-l-}* (black circles) animals. Quantification of tumor growth which correlates to photon flux (p/s) in the region of interest (ROI) versus the days post injection of the modified glioma cells are shown (upper panel). Lower panel: Kaplan-Meier survival analysis. Data are representative of 2 independent experiments.

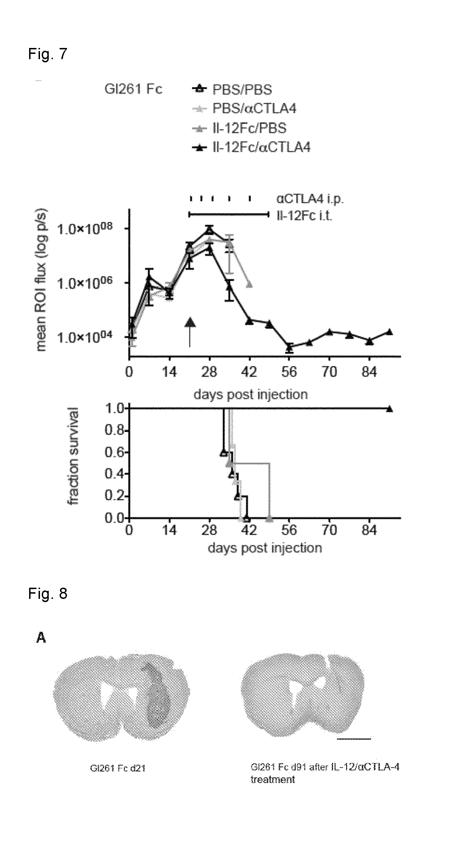

- Fig. 7: shows tumor growth in wt mice inoculated with 2x10⁴ GI261 Fc cells. Treatment started at day 21 (arrows). Osmotic minipumps delivering IL-12Fc (or PBS) into the tumor were implanted into glioma bearing animals. Animals received i.p. injections of αCTLA-4 blocking antibodies or PBS starting at day 22, followed by injections as indicated in figure. Upper graph: quantification of ROI photon flux of tumor bearing wt animals receiving the indicated treatment. Lower graph: Kaplan-Meier survival analysis of the animals above; PBS/PBS vs IL-12/αCTLA-4 p=0.0045, PBS/PBS vs IL-12/PBS p=0.3435, PBS/ αCTLA4 vs IL-12/αCTLA-4 p=0.0101; Log- rank (Mantel-Cox) Test. Data representative of three independent experiments with 2-4 animals per group
- Fig. 8: shows immunohistochemistry of tumor sections obtained from syngenic C57/BI6 mice after challenge with GI261 Fc cells at day 21 and after local administration of IL-12Fc in combination with systemic CTLA-4 blockade as described in Example 5. The sections were stained with Hematoxylin and Eosin. Scale bar indicates 2 mm.

### Examples

### Methods

### Animals

C57BL/6 mice were obtained from Janvier; *b2m*^{*-*/}*⁻, la(b)^{-l-}, II12rb2*^{*-*/}*⁻, Rag1*^{*-*/}*⁻, Rag2*^{*-*/}*⁻II2rg*^{*-*/}*⁻, Prf1*^{*-*/*-*} and *Ifng*^{-/-}mice were obtained from Jackson Laboratories. *II15ra*^{-/-}mice were provided by S. Bulfone-Paus. All animals were kept in house under specific pathogen-free conditions at a 12hour light/dark cycle with food and water provided *ad libitum.* All animal experiments were approved by the Swiss Cantonary veterinary office (16/2009).

### Mouse glioma ce// lines

C57/BI6 murine glioma (GI261) cells (kindly provided by A. Fontana, Experimental Immunology, University of Zürich) were transfected with pG13-ctrl (Promega) and pGK-Puro (kindly provided by T. Buch, Technical University Munich). Linearized constructs were electroporated in a 10:1 1 ratio using an eppendorf multiporator, then selected with 0.8µg/ml puromycin (Sigma-Aldrich) to generate luciferase-stable GI261 cells. A single clone was isolated by limiting dilution and passaged in vivo by intracranial tumor inoculation, followed by tumor dissociation after 4 weeks and re-selection in 0.8µg/ml puromycin. Subsequently, cells were electroporated with pCEP4-mIgG3, pCEP4-mll-12mIgG3 and pCEP4-mII-23mIgG3 (Eisenring et al, 2010) and bulk-selected with 0.8pg/mIpuromycin and 0.23mg/ml hygromycin (Sigma-Aldrich). Cytokine production was detetected by ELISA (OptEIA II-12/23p40, BD Pharmingen) and rt-PCR (IgG3fw: ACACACAGCCTGGACGC (SEQ ID 03) IgG3rev: CATTTGAACTCCTTGCCCCT (SEQ ID 04)). GI261 cells and derived cell lines were maintained in Dulbecco's modified Eagle's medium (Gibco, Invitrogen) supplemented with 10% fetal calf serum (FCS) in presence of selection antibiotics as indicated above at 37°C and 10% CO₂.

### Expression and purification of IL-12Fc

IL-12Fc was expressed in 293T cells after calcium phosphate-mediated transfection according to standard protocols with 45µg of vector DNA (pCEP4-mIL-12IgG3)/15cm tissue culture plate. Supernatant was harvested 3 days and 6 days after transfection, sterile filtered and diluted 1:1 in PBS. The protein was purified using a purifier (ÄktaPrime) over a protein G column (1ml, HiTrap, GE Healthcare) eluted with 0.1 M glycine pH 2 and dialyzed over night in PBS pH 7.4. Concentration and purity of IL-12Fc was measured by ELISA (OptEIA 11-12/23p40, BD Pharmingen) and SDS-PAGE followed by silverstaining and immunoblotting. IL-12Fc was detected with a rat anti mouse IL-12p40 antibody (C17.8, BioExpress) and a goat anti-rat HRP coupled antibody (Jackson).

### Orthotopic glioma inoculation

Briefly, 6-10 week old mice were i.p. injected with Fluniximin (Biokema, 5mg/kg body weight) before being anaesthesized with 3-5% isoflurane (Minrad) in an induction chamber. Their heads were shaved with an electric hair-trimmer. After being mounted onto a stereotactic frame (David Kopf Instruments), the animals' scalp was disinfected with 10% iodine solution and a skin incision was made along the midline. Anaesthesia on the stereotactic frame was maintained at 3% isoflurane delivered through a nose adaptor (David Kopf Instruments). Subsequently, a blunt ended syringe (Hamilton, 75N, 26s/2"/2, 5µl) was mounted on a microinjection pump on the manipulator arm and placed 1.5mm lateral and 1 mm frontal of bregma. The needle was lowered into the manually drilled burr hole at a depth of 4mm below the dura surface and retracted 1 mm to form a small reservoir. Using the microinjection pump (UMP-3, World Precision Instruments Inc.) 2x10⁴ cells were injected in a volume of 2µl at 1 µl/min. After leaving the needle in place for 2min, it was retracted at 1 mm/min. The burr hole was closed with bone wax (Aesculap, Braun) and the scalp wound was sealed with tissue glue (Indermil, Henkel).

### In vivo bioluminescent imaging

Tumor bearing mice were carefully weighed, anaesthesized with isoflurane (2-3%) and injected with D-Luciferin (150mg/kg body weight, CaliperLifesciences). Animals were transferred to the dark chamber of a Xenogen IVIS 100 (CaliperLifesciences) imaging system, anaesthesia was maintained at 2% isoflurane via nosecones. 10min after injection luminescence was recorded. Data was subsequently analyzed using Living Image 2.5 software (CaliperLifesciences). A circular region of interest (ROI; 1.46cm 0) was defined around the animals' head and photon flux of this region was read out and plotted.

### Treatment of established gliomas

At d21 after implantation of the glioma cells, the tumor bearing animals were evenly distributed among experimental groups based on their ROI-photon flux. Animals with an ROI flux of less than 1x10⁵ p/s were considered as non-takers and excluded. 40-48h prior to implantation (2 days before beginning of treatment), osmotic pumps (Model 2004, 0.25µl/h; Alzet) were filled with murine IL-12Fc (8.33 ng/µl in PBS) or PBS alone and primed at 37°C in PBS. Immediately prior to surgery, mice were injected with Fluniximini.p. (Biokema, 5mg/kg body weight). Mice were anaesthesized with 3-5% isoflurane, the scalp was disinfected and a midline incision was made. The previous burr hole of the glioma injection was located, the bone wax and periost removed and the pump placed into a skin pouch formed at the animal's back. The infusion cannula was lowered through the burr hole 3mm into the putative center of the tumor. The cannula was connected to the pump (brain infusion kit III 1-3mm, Alzet) via a silicon tube and held in place with cyanoacrylate adhesive. The skin was sutured with a 4-0 nylon thread. Following surgery, mice were treated for 3 days with 0.1 % (v/v) Borgal (Intervet) in the drinking water. Pumps were explanted at day 49. Five doses of anti mouse-CTLA-4 mouse-IgG2b antibodies (clone 9D9, bio-X-cell; Peggs et al.;J Exp Med 206, 1717-1725 (2009)) or an equivalent volume of PBS were i.p. injected at days 22 (200µg), 26 (100µg), 29 (100µg), 35 (100µg) and 42 (100µg). For systemic treatment, animals received 200µg anti mouse-CTLA-4 and/or 200ng rIL-12 (Peprotech) in PBS starting at day 21 via i.p. injection. Treatment was sustained with 100µg anti mouse-CTLA-4 and/or 100ng rIL-12 three times per week until the end of the experiment.

### Survival analysis of tumor bearing animals

Tumor bearing animals were monitored by BLI, checked for neurological symptoms and weighed weekly until day 21 post glioma inoculation. GI261 Fc animals exhibiting an ROI flux of less than 1x10⁵ p/s at day 21 were considered as non or slow-tumor takers and excluded from the survival analysis (5-10%). From day 21 onwards animals were checked daily. Animals that showed symptoms as apathy, severe hunchback posture and/or weight loss of over 20% of peak weight were euthanized.

### Histology

For histology, animals were euthanized with CO₂, transcardially perfused with ice-cold PBS and decapitated. Whole brains were carefully isolated, fixed in 4% Formalin, embedded in Paraffin and 3µm sections were processed for HE staining and/or immunohistochemistry to detect F4/80 (BMB; BMA biomedicals). Primary antibodies were detected with HorseRadish Peroxidase- coupled secondary antibodies. Staining was visualized with 3,3'-Diaminobenzidin (DAB) as the HRP substrate. Pictures were generated using an Olympus BX41 light microscope equipped with an Olympus ColorViewIIIu camera and Olympus cell^B image acquisition software. Overviews of whole brains slices were cropped using Adobe Photoshop CS3.

### Statistical analysis

For statistical analysis of Kaplan-Meier survival curves, a Log-rank (Mantel-Cox) Test was used to calculate the p-values indicated in respective figures. P values of less than 0.05 were considered statistically significant. Analysis was performed with GraphPad Prism version 5.0a for Mac OSX (GraphPad Software Inc).

### Example 1: Intratumoral expression of IL-12Fc promotes clearance of experimental gliomas

To determine if IL-12Fc is suitable to overcome the local immunosuppressive environment induced by gliomas and to shed light on the effector mechanisms involved, we expressed this cytokine in GI261 mouse glioma cells. To measure intracranial tumor growth non-invasively via bioluminescence imaging (BLI) we first generated a GI261 line that constitutively expresses *photinus pyralis* luciferase. We termed this cell line GI261-luc. We next modified this cell line to continuously release a fusion protein of IL-12 and the crystallizable fragment of mouse immunoglobulin G3 (IL-12Fc, SEQ ID 02) or Fc alone as a control (termed 'GI261 IL-12Fc'and 'GI261 Fc', respectively). The chosen murine protein sequence of the fusion construct is homologous to the human variant (SEQ ID 01) and consists of the subunits p40 and p35 of IL-12 which are connected by a linker (G4S)₃ and the subunits CH2, CH3 and the last six amino acids of CH1 of the crystallizable fragment of IgG3, whereas CH1 and CH2 are connected by a hinge region. Vectors for expression of the control fragment and the IL-12 fusion construct are depicted in SEQ ID 08 and 09, respectively. The intention of using fusion proteins rather than the recombinant cytokine was to see whether they would exhibit improved pharmacokinetics. We confirmed secretion of IL-12Fc by ELISA for the subunits p40 and p70. We further confirmed expression of the Fc tail by RT-PCR. When implanted intracranially into the right striatum, luminescence readings and tumor volume as assessed by stereologic methods showed a robust correlation (data not shown).

We next implanted GI261 IL-12Fc and Fc into the right striatum of syngenic C57BI/6 mice and followed tumor growth via non invasive bioluminescence imaging (BLI). After an initial increase in luminescence all groups showed a depression around day 14 post injection. Animals bearing Fc-expressing tumors exhibited a steep increase in BLI and soon reached withdrawal criteria, sometimes even before day 35 post injection. In contrast, BLI-readings for animals that had been injected with IL-12Fc expressing GI261 tumors dropped to levels close to the detection limit at day 21 onwards (data not shown). In agreement with this observation, we could only detect a residual tumor in some animals in this group, while Fc control-injected animals showed robust tumor formation when analyzed histologically (Fig. 2). When we followed animals that had been implanted with GI261 IL-12Fc or GI261 Fc cells for up to 90 days, we observed rejection of the tumor in a high proportion of mice bearing IL-12Fc secreting tumors after an initial establishment ( Fig.).

### Example 2: T-cells are the major effector cell type of IL-12Fc mediated glioma rejection.

To confirm that the secretion of IL-12Fc by GI261 IL-12Fc acts on the host rather than the tumor cells themselves, we observed the growth of GI261 IL-12Fc and G1261-Fc to be the same in mice lacking the receptor to IL-12. The unbridled growth of GI261 IL-12 in *IL-12rβ2*^{*-*/*-*} animals demonstrates that IL-12Fc acts specifically on a cell type in the recipient mouse (data not shown). T and NK cells are among the most prominent IL-12 responsive leukocytes. To systematically test the functional relevance of the IL-12Fc mediated influx of these cells, we challenged a series of mouse mutants with intracranial GI261 IL-12Fc. We implanted GI261 IL-12Fc cells in mice that lack T and B cells (Rag1^{-/-}) or conventional Nk-cells *(II-15ra*^{*-*/}*⁻)* or in mice lacking both T-, B-, Nk-cells and lymphoid tissue inducer-like cells *(Rag2*^{*-*/}*⁻ II2rg*^{*-*/}*⁻)* (Fig.A). After an initial lag phase until day 14 after injection, all groups exhibited a strong increase in luminescence until day 28, reflecting strong tumor growth. Between days 28 and 42 most of the animals succumbed to the tumors. Only wt and *II-15ra^{-l-}* mice were able to control the tumor and show a significantly prolonged survival compared to *Rag2*^{*-*/}*⁻ II2rg^{-l-}* and *Rag1*^{*-*/*-*} animals. While T or B-cells appeared to be crucial for IL-12Fc mediated glioma rejection, the ability of *II-15ra^{-l-}* mice to reject GI261 IL-12 indicates that NK cells were largely expendable.

We next investigated the contribution of CD4- and CD8 positive T-cells using MHCII *(Ia(b)^{-l-})* and MHCI (*β2m*^{-/-}) deficient mice. In contrast to wt mice, *Ib(b)*^{*-*/*-*} mice lacking CD4 T cells could not control GI261 IL-12Fc tumors, and *β2m*^{*-*/*-*} mice succumbed to the glioma shortly afterwards (Fig.B).The survival in both mutant groups was shortened compared to the wildtype group. These data clearly demonstrate that IL-12Fc mediated tumor rejection is dependent on the activity of T cells including helper T cells and CTLs.

### Example 3: The anti-tumoral memory response is independent of ectopically expressed IL-12Fc.

To further investigate the character of the T-cell dependent tumor control, we tested the surviving wt animals that had been previously challenged with GI261 IL-12Fc cells for T cell memory formation (Fig. 5). The animals were treated as described in Fig. 3 / example 1. In contrast to the primary challenge, we now injected GI261 Fc cells into the contralateral hemisphere of survivors or naïve wt animals. We observed a rapid rejection of the control tumors within days. While the measured luminescence at day 1 suggested identical seeding across the two groups, only the naïve mice exhibited a measurable signal at day 7 onwards, suggesting a rapid and effectively clearing anti-glioma memory response now independent of ectopically expressed pro-inflammatory cytokines.

### Example 4: CTLs are the main effector cells of IL-12Fc-mediated glioma rejection.

It is well established that IL-12 polarizes naive T-cells to adopt a T_{H}1 phenotype (Trinchieri, Nat Rev Immunol 3, 133-146 (2003)). To shed further light on the mechanistic underpinnings underlying the IL-12 induced rejection of experimental glioma, we challenged mice deficient in the T_{H}1 hallmark cytokine IFN-y (IFN- *_{Y}*^{-/-}) with IL-12Fc expressing GI261 cells (Fig.A). The animals were treated as described in Fig. 3/ Example 1. To our surprise we observed a similar tumor rejection as in wt animals, suggesting that the mechanism of rejection is independent of IFN-y. Conversely, IL-12 also stimulates the cytotoxic activity of CTLs. When we analyzed the role of Perforin, a cytolytic molecule primarily expressed on CD8⁺ CTLs and Nk-cells, we observed a clear difference in survival curves. (Fig. 6 B). Perforin is a cytolytic molecule primarily expressed on CD8⁺ CTLs and NK cells. To further investigate the mechanism of IL-12Fc induced rejection of glioma, perforin-deficient mice (prf1^{-/-}) were challenged with IL-12Fc expressing GI261 cells.In contrast to *IFN-_{Y}*^{*-*/-}, Perforin deficient animals (*prf1*^{*-*/*-*}) were not able to control the tumor. This further supports the notion that CTLs are the main effector cells of IL-12Fc mediated glioma rejection. A clear difference in the survival curves of wt and *prf1*^{-/-}was observed.

### Example 5: Local administration of IL-12Fc in combination with systemic CTLA-4 blockade is effective against advanced stage experimental gliomas

To further boost and prolong the activated phenotype of T-cells, we blocked the co-inhibitory molecule CTLA-4 via neutralizing antibodies in the next set of experiments. IL-12 was administered locally to mice with advanced stage tumors. Treatment was administered to animals that had been challenged with GI261 Fc 21 days before and that already exhibited strong bioluminescence signals, indicating an advanced stage of glioma growth. Local treatment: At day 21, osmotic minipumps delivering 50 ng IL-12Fc/day (or PBS) into the tumor were implanted into glioma bearing animals. After 28 days (day 49 after tumor injection) the empty pumps were explanted from surviving animals. Systemic treatment: At day 22, tumor bearing animals received 200 µg αCTLA-4 mouse IgG2b (9D9) or PBS i.p. Treatment was sustained with 100 µg αCTLA-4 at days 26, 29,35 and 42 (Fig 7). Neither IL-12Fc, nor anti-CTLA-4 alone conferred any significant survival advantage. Strikingly, the combination of local IL-12Fc administration directly into the tumor site in combination with systemic CTLA-4 blockade led to a full remission of the tumor (Fig.). 90 days after inoculation, histologic assessment of the brain tissue of surviving animals did not show any signs of demyelination or infiltrates.

Preventive treatment of tumors in preclinical models may allow the study of immunological mechanisms and the interactions between tumor cells and tumor microenvironment. However, preventive therapy is of little to no clinical relevance in the translation to treat cancer patients. We thus decided to choose an exceptionally late timepoint for intervention in a progressing and aggressive disease model. To closely mimic a clinical situation, we allowed the tumor to progress to a size that is highly likely to cause significant neurological symptoms in humans. Here, monotherapy with locally applied (intratumoral) IL-12 had a minimal albeit significant survival effect. We already observed a weak synergistic effect when we combined systemic IL-12 treatment with systemic CTLA-4 blockade. When local IL-12 infusion was combined with systemic CTLA-4 blockade, the anti-glioma effect was striking.

## Claims

1. A combination medicament, comprising
- an IL-12 polypeptide, or a nucleic acid expression vector comprising a sequence encoding said IL-12 polypeptide, and
- a non-agonist CTLA-4 ligand.

2. A combination medicament according to claim 1, wherein said IL-12 polypeptide comprises
a. a polypeptide sequence at least 95% identical to the sequence of human p35 (SEQ ID 05), and
b. a polypeptide sequence at least 95% identical to the sequence of human p40 (SEQ ID 06).

3. A combination medicament according to claim 2, wherein said IL-12 polypeptide is a recombinant or synthetic human IL-12 or a polypeptide comprising a sequence at least 95% identical to SEQ ID 01.

4. A combination medicament according to any of the above claims, wherein said non-agonist CTLA-4 ligand is a gamma immunoglobulin that binds to CTLA-4.

5. A combination medicament according to any of the above claims, wherein said IL-12 polypeptide comprises an immunoglobulin G crystallisable fragment.

6. A combination medicament according to claim 5, wherein said immunoglobulin G crystallisable fragment is a human immunoglobulin G subgroup 4 crystallisable fragment.

7. A combination medicament according to any of claims 1 to 6, wherein said IL-12 polypeptide is provided as a dosage form for intratumoral injection, and said non-agonist CTLA-4 ligand is provided as a dosage form for intravenous injection.

8. A combination medicament according to at least one of claims 1 to 5 for therapy of a malignant neoplastic disease.

9. A combination medicament according to at least one of claims 1 to 5 for therapy of glioma, glioblastoma multiforme, meningioma, melanoma, pancreatic cancer, lung cancer, prostate cancer or bladder cancer.

10. A combination medicament according to claim 1, wherein said IL-12 polypeptide having a biological activity of IL-12 is a fusion protein comprising the amino acid of human p40, the amino acid sequence of human p35 and the crystallisable fragment of human IgG4 provided as a dosage form for intratumoural delivery, and wherein said non-agonist CTLA-4 ligand is a immunoglobulin G provided as a dosage form for systemic delivery, for the treatment of malignant neoplastic disease particularly glioma, glioblastoma multiforme, meningioma, melanoma, pancreatic cancer, lung cancer, prostate cancer or bladder cancer.

11. A combination medicament according to at least one of claims 1 to 7, wherein said nucleic acid expression vector is an adenovirus, an adeno-associated virus, a lentivirus or a herpesvirus.

12. Use of an IL-12 polypeptide having a biological activity of IL-12, and a non-agonist CTLA-4 ligand in the manufacture of a combination medicament for therapy of a malignant neoplastic disease, glioma, glioblastoma multiforme, meningioma, melanoma, pancreatic cancer, lung cancer, prostate cancer or bladder cancer.

13. A method of treating a patient suffering from malignant neoplastic disease, comprising the administration of an IL-12 polypeptide having a biological activity of IL-12, and a non-agonist CTLA-4 ligand.
